# EUROPEAN PATENT SPECIFICATION

(11) **EP 3 329 934 B1**
(45) Date of publication and mention of the grant of the patent: **24.03.2021**
(21) Application number: 16842279.8
(22) Date of filing: 31.08.2016
(51) Int. Cl.: A61K 38/20, A61K 39/235, A61K 48/00, A61P 35/00

(54) **ANTITUMOR IMMUNITY ENHANCING COMPOSITION CONTAINING ADENOVIRUS SIMULTANEOUSLY EXPRESSING IL-12 AND SHVEGF**
ZUSAMMENSETZUNG ZUR ERHÖHUNG DER ANTITUMORIMMUNITÄT MIT ADENOVIRUS ZUR GLEICHZEITIGEN EXPRESSION VON IL-12 UND SHVEGF
COMPOSITION AMÉLIORANT L'IMMUNITÉ ANTITUMORALE CONTENANT L'ADÉNOVIRUS EXPRIMANT SIMULTANÉMENT IL-12 ET SHVEGF

(30) Priority: 01.09.2015 KR 20150123644
(43) Date of publication of application: 06.06.2018
(73) Proprietor: Genemedicine Co., Ltd., Seongdong-gu Seoul 04763 (KR)
(72) Inventor: YUN, Chae Ok, Seoul 06583 (KR); AHN, Hyo Min, Incheon 22730 (KR)
(74) Representative: Prüfer & Partner mbB Patentanwälte · Rechtsanwälte
(86) International application number: PCT/KR2016/009717
(87) International publication number: WO 2017/039313

(56) References cited:
- KR-A- 20070 042 283
- KR-A- 20090 007 067
- KHAN W I ET AL: "IL-12 gene transfer alters gut physiology and host immunity in nematode-infected mice", AMERICAN JOURNAL OF PHYSIOLOGY - GASTROINTESTINAL AND LIVER PHYSIOLOGY, AMERICAN PHYSIOLOGICAL SOCIETY, US, vol. 281, no. 1, 30 June 2001 (2001-06-30) , pages G102-G110, XP009505602, ISSN: 0193-1857
- LIANG WEN ET AL: "In vitro induction of specific anti-tumoral immunity against laryngeal carcinoma by using human interleukin-12 gene-transfected dendritic cells", CHINESE MEDICAL JOURNAL / ZHONGHUA YIXUE ZAZHI YINGWEN BAN, CHINESE MEDICAL ASSOCIATION, BEIJING, CN, vol. 124, no. 9, 30 April 2011 (2011-04-30), pages 1357-1361, XP009505599, ISSN: 0366-6999
- CHEN S H ET AL: "Rejection of disseminated metastases of colon carcinoma by synergism of IL-12 gene therapy and 4-1BB costimulation", MOLECULAR THERAPY : THE JOURNAL OF THE AMERICAN SOCIETY OF GENE THERAPY, ACADEMIC PRESS ; NATURE PUBLISHING GROUP, US, vol. 2, no. 1, 30 June 2000 (2000-06-30), pages 39-46, XP009505601, ISSN: 1525-0016
- RODRIGO GARZÓN MANUEL ET AL: "[Use of gene therapy in a subcutaneous murine model of lung cancer]", ARCHIVOS DE BRONCONEUMOLOGIA, DOYMA, BARCELONA, ES, vol. 42, no. 10, 30 September 2006 (2006-09-30), pages 526-532, XP009505600, ISSN: 0300-2896, DOI: 10.1016/S1579-2129(06)60580-6
- CARUSO M ET AL: "ADENOVIRUS-MEDIATED INTERLEUKIN-12 GENE THERAPY FOR METASTATIC COLON CARCINOMA", PROCEEDINGS OF THE NATIONAL ACADEMY OF SCIENCES, NATIONAL ACADEMY OF SCIENCES, US, vol. 93, 1 January 1996 (1996-01-01), pages 11302-11306, XP000887255, ISSN: 0027-8424, DOI: 10.1073/PNAS.93.21.11302
- SIOBHAN M CASHMAN ET AL: "Inhibition of Choroidal Neovascularization by Adenovirus-Mediated Delivery of Short Hairpin RNAs Targeting VEGF as a Potential Therapy for AMD", INVESTIGATIVE OPHTHALMOLOGY & VISUAL SCIENCE, vol. 47, no. 8, 1 August 2006 (2006-08-01) , pages 3496-3504, XP55289337, DOI: 10.1167/iovs.05-1610
- DATABASE WPI Week 201004 Thomson Scientific, London, GB; AN 2010-A23395 XP002781873, & CN 101 596 316 A (CHANGSHA XINSHENG KANGYUAN BIOPHARMACEUTICAL CO LTD) 9 December 2009 (2009-12-09)
- LEE ET AL.: 'Enhanced Antitumor Effect of Oncolytic Adenovirus Expressing Interleukin-12 and B7-1 in an Immunocompetent Murine Model' CLINICAL CANCER RESEARCH vol. 12, no. 19, 2006, pages 5859 - 5868, XP055057666
- TUGUES ET AL.: 'New Insights into IL-12-mediated Tumor Suppression' CELL DEATH AND DIFFERENTIATION vol. 22, no. 2, February 2015, pages 237 - 246, XP055285716
- YOO ET AL.: 'Short Hairpin RNA-expressing Oncolytic Adenovirus-mediated Inhibition of IL-8: Effects on Antiangiogenesis and Tumor Growth Inhibition' GENE THERAPY, VOL. vol. 15, 2008, pages 635 - 651, XP055376224

## Description

### [Technical Field]

The present invention relates to a composition for enhancing antitumor immunity including adenovirus co-expressing IL-12 and shVEGF.

### [Background Art]

Immunological dysfunction in cancer patients has been well known [1], and it is also clinically apparent that tumors effectively avoid anticancer immune responses. While recent anticancer treatment mainly focuses on surgical operations, chemotherapy and radiation therapy, these are all accompanied by side effects [2, 3]. To overcome such barriers and increase the efficiency of anticancer treatment, research on immunotherapy is actively progressing. In the last decades, because of new findings in basic immunology and molecular discoveries of tumor antigens, research on immunotherapies for preventing and treating cancer have been actively conducted [4]. Tumor cells express a great quantity of abnormal antigens by themselves, and these antigens cause an eradication response through immune surveillance. Even if the immune system in the body actively acts, the tumor cells can avoid immune surveillance [5, 6]. It was identified that such a phenomenon is mediated by various factors produced by tumor cells. Tumor tissues can produce immunosuppressive molecules such as a vascular endothelial growth factor (VEGF), tumor growth factor (TGF)-β and interleukin (IL)-10 [7, 8]. It has also been reported that infiltration of regulatory T cells occurs in immunosuppressed tumors [9-11]. These factors that are overexpressed in tumor tissue induce an immunosuppressive microenvironment and immune tolerance in tumors. As a result, overcoming immune surveillance is a pivotal strategy in immunotherapy.

IL-12 is one of the most effective and promising anticancer cytokines. IL-12 is a heterodimeric protein including 40 kDa and 35 kDa subunits connected by a disulfide bond, and produced by activated macrophages, monocytes, dendritic cells and active B lymphocytes. IL-12 may enhance T-helper 1 (Th1) cell immunity [12] and the cytotoxicity of cytotoxic T-lymphocytes, and suppress angiogenesis. Generally, IL-12 activates IFN-γ production of T cells and natural killer (NK) cells [13]. Local expression of IL-12 allows tumor cells to sensitively response to T-cell mediated cytotoxicity, resulting in tumor growth inhibition and the establishment of systemic immunity. However, a disadvantage of the clinical application of IL-12 is that cytokine-associated toxicity that limits the acceptable dose for a patient when administered may appear in the entire body [14-17]. In addition, overall downregulation of the immune effect may result in repeated administration of IL-12. In a patient's serum, IL-12 polarization from Th1 to Th2 immunity occurs due to an increase in IL-10 expression and a decrease in IFN-γ and TNF-β expression, and for this reason, IL-12 is repeatedly administered [18,19]. These clinical results show the limitations of IL-12 as a single therapeutic for cancer treatment.

VEGF is a signal protein produced by cells, stimulating vasculogenesis and angiogenesis [20]. Particularly, VEGF affects T cell precursors in bone marrow and thus inhibits the proliferation and maturation of T cells and dendritic cells [21]. In addition, VEGF plays an important role in angiogenesis and represents a side effect called metastasis [22]. Therefore, VEGF is a stimulating factor of tumor growth, and also acts as an inhibitory factor in anticancer immunity, and it is expected that VEGF downregulation caused by VEGF shRNA will restore immune responses and increase anticancer effects. These studies show that the therapeutic mechanism mediated by VEGF shRNA, similar to that by IL-12, is associated with enhancement in CTL or dendritic cell-mediated immune responses.
refers to the administration of recombinant adenovirus vector expressing IL-12 (Ad5IL-12) for altering the gut physiology and host immunity in the treatment of mice infected with nematodes.
refers to the in-vitro induction of anti-tumoral immunity against laryngeal carcinoma by using IL-12 gene-transfected dendritic cells, including also the use of constructions comprising recombinant adenovirus with IL-12 gene.
concerns the synergism of IL-12 gene therapy and 4-1BB costimulation in the rejection of disseminated metastases of colon carcinoma, whereby vector constructions comprising recombinant adenovirus expressing IL 12 replacing the E1 A viral region are used.
refers to the use of gene therapy in a lung cancer tumor, reporting on the efficient use of AdCMV-IL-12 in the reduction of subcutaneous tumor growth.
refers to the use of adenovirus-mediated interleukin-12 gene therapy for metastatic colon carcinoma.
refers to the inhibition of choroidal neovascularization by adenovirus-mediated delivery of shRNA targeting VEGF as potential therapy for AMD.
discloses the method of preventing or treating tumor by providing a recombinant virus expressing shRNA to inhibit FoxM1.
discloses a recombinant adenovirus having improved oncolytic activity and expressing VEGF specific siRNA complementary to an mRNA of VEGF-A of SEQ ID NO: 1.

Therefore, the inventors suggest a method for more effectively increasing antitumor immunity using a combination of the above-mentioned immunotherapies.

A variety of papers and patent documents are referred throughout the specification, and their citations are indicated. The disclosures of the cited papers and patent documents describe the standard of the field of the art to which the present invention belongs and the scope of the present invention.

### [Disclosure]

### [Technical Problem]

The inventors had attempted to develop immunotherapy for overcoming the avoidance of immune surveillance of tumors using an immunosuppressive molecule produced in tumor tissue. As a result, the inventors constructed an adenovirus co-expressing IL-12 and shVEGF and developed a composition for improving immunity and a composition for improving an anticancer effect, which include the adenovirus. In addition, the inventors identified that a therapeutic effect can be enhanced by applying such an adenovirus (RdB/IL12/shVEGF) co-expressing IL-12 and shVEGF to cancer therapy, and thus completed the present invention.

Therefore, the present invention is directed to providing a recombinant adenovirus, comprising (i) an interleukin (IL-12) gene having at least one of an IL-12A (p35) gene sequence and an IL-12B (p40) gene sequence, and (ii) a small hairpin RNA (shRNA) gene which is complementary to VEGF mRNA, and suppresses VEGF gene expression.

The present invention further refers to a composition for use in a method of enhancing antitumor immunity, comprising (a) a therapeutically effective amount of a recombinant adenovirus comprising (i) an interleukin (IL-12) gene having at least one of an IL-12A (p35) gene sequence and an IL-12B (p40) gene sequence, and (ii) a small hairpin RNA (shRNA) gene which is complementary to VEGF mRNA, and suppresses VEGF gene expression; and (b) a pharmaceutically acceptable carrier.

The present invention further refers to an anticancer composition, comprising (a) a therapeutically effective amount of a recombinant adenovirus comprising (i) an interleukin (IL-12) gene having at least one of an IL-12A (p35) gene sequence and an IL-12B (p40) gene sequence, and (ii) a small hairpin RNA (shRNA) gene which is complementary to VEGF mRNA, and suppresses VEGF gene expression; and (b) a pharmaceutically acceptable carrier, and to said anticancer composition for use in a method of treating specific cancer.

Finally, the present invention refers to a composition for use in a method of preventing or treating tumor-induced thymic atrophy, comprising (a) a therapeutically effective amount of a recombinant adenovirus comprising (i) an interleukin (IL-12) gene having at least one of an IL-12A (p35) gene sequence and an IL-12B (p40) gene, and (ii) a small hairpin RNA (shRNA) gene which is complementary to VEGF mRNA, and suppresses VEGF gene expression; and (b) a pharmaceutically acceptable carrier.

Other objects and advantages of the present invention will become more apparent by the detailed description of the invention, claims and drawings as follows.

### [Technical Solution]

According to an aspect of the present invention, the present invention provides a recombinant adenovirus, comprising (i) an interleukin (IL-12) gene having at least one of an IL-12A (p35) gene sequence and an IL-12B (p40) gene sequence, and (ii) a small hairpin RNA (shRNA) gene which is complementary to VEGF mRNA, and suppresses VEGF gene expression,
, or a composition for use in a method of enhancing antitumor immunity, comprising (a) a therapeutically effective amount of a recombinant adenovirus comprising (i) an interleukin (IL-12) gene having at least one of an IL-12A (p35) gene sequence and an IL-12B (p40) gene sequence, and (ii) a small hairpin RNA (shRNA) gene which is complementary to VEGF mRNA, and suppresses VEGF gene expression; and (b) a pharmaceutically acceptable carrier.

Upon conducting the research to develop immunotherapy to overcome the avoidance of immune surveillance of tumors using an immunosuppressive molecule produced in tumor tissue, the inventors constructed an adenovirus co-expressing IL-12 and shVEGF, and developed a composition for enhancing antitumor immunity and a composition for enhancing an anticancer effect. The inventors identified that a therapeutic effect can be improved by applying such an adenovirus co-expressing IL-12 and shVEGF (RdB/IL12/shVEGF) to gene therapy for cancer.

Cancer immunogenic therapy has been developed to be a more promising approach over the last few decades. However, to avoid the immune surveillance system, a tumor also has a variety of different strategies. To overcome such a barrier and enhance an anticancer immunity effect, a system (RdB/shVEGF) co-expressing IL-12 (RdB/IL12) and VEGF shRNA and an oncolytic co-expression adenovirus (RdB/IL12/shVEGF) system thereof were constructed as proper therapeutic adjuvants for restoring immunosuppression and increasing anticancer immunity. IL-12 induces the differentiation of Th1 cells and activates cytotoxic T lymphocytes and the cytotoxicity of NK cells, thereby increasing anticancer immunity. shVEGF is very effective for inhibiting VEGF expression, and thus stimulates the proliferation and maturation of T cells and dendritic cells. Such effects further amplify the functionality of IL-12. However, there is no research on the therapeutic effect caused by the co-expression of IL-2 and shVEGF in tumors. Therefore, the inventors first studied an effect of immunogenic therapy of injecting an adenovirus (RdB/IL12/shVEGF) co-expressing IL-2 and shVEGF into a tumor. In a B16-F10 murine melanoma model, the injection of RdB/IL12/shVEGF into a tumor greatly improves an anticancer effect. While IL-12 and IFN-γ expression levels were considerably increased in the RdB/IL12/shVEGF-treated group, a VEGF expression level was decreased. In addition, a ratio of T-helper type 1/2 cytokines was increased in splenocyte-and-B16-F10 co-cultured supernatants. The cytotoxicity of cancer-specific immune cells in RdB/IL12/shVEGF-injected mice was increased. Like the above-described result, in situ delivery of RdB/IL12/shVEGF results in a phenomenon of massive infiltration of CD4⁺ T cells, CD8⁺ T cells, NK cells and CD86⁺ APCs into tissues surrounding the necrotic region of a tumor. Importantly, the adenovirus co-expressing IL-12 and shVEGF prevents tumor-induced thymic atrophy, is associated with a reduction in cell death, and increases cell proliferation in the thymus. An oncolytic adenovirus co-expressing IL-12 and shVEGF can be used as an adjuvant that increases an anticancer effect, and enhances antitumor immunity.

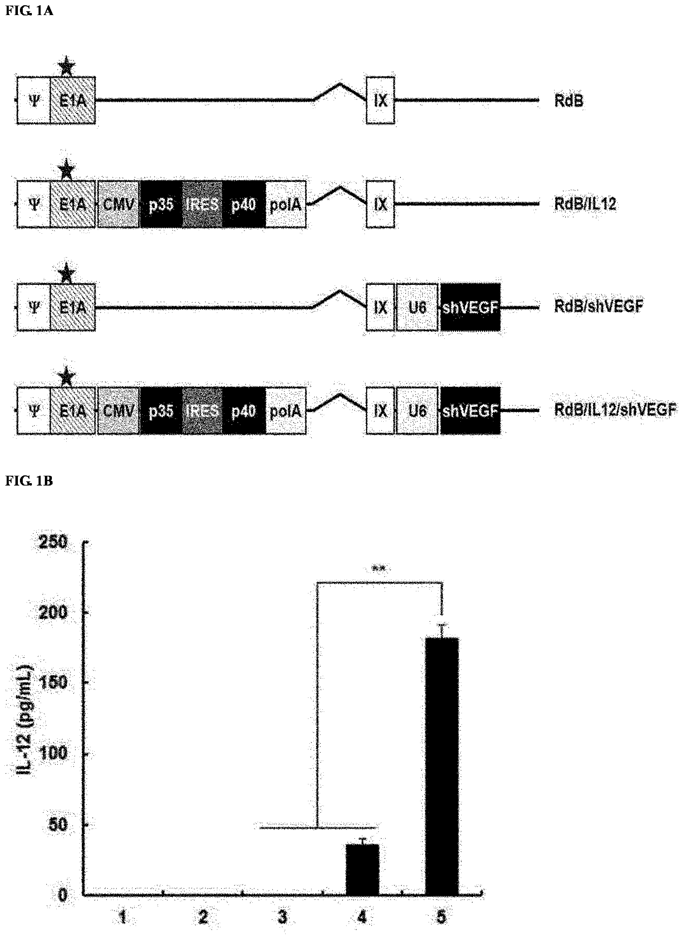

The recombinant adenovirus of the present invention includes both of an IL-12 gene sequence and a vascular endothelial growth factor small hairpin RNA (shVEGF) gene sequence. The IL-12 gene sequence includes at least one of an IL-12A (p35) gene sequence and an IL-12B (p40) gene sequence, and according to an exemplary embodiment of the present invention, the IL-12 gene sequence includes an IL-12A (p35) gene sequence, an internal ribosome entry site (IRES) sequence and an IL-12B (p40) gene sequence (refer to FIG. 1A, RdB/IL12 and RdB/IL12/shVEGF).

It should be interpreted that the IL-12 gene sequence used in the present invention also includes a gene sequence exhibiting substantial identity or substantial similarity to the sequence of at least one of SEQ ID NO: 1 and SEQ ID NO: 2. The substantial identity refers to a sequence having at least 80% homology, more preferably 90% homology, and most preferably 95% homology, when a random sequence is aligned to correspond as much as possible with the sequence of the present invention, and the aligned sequences are analyzed using an algorithm conventionally used in the art. The substantial similarity encompasses all of the changes in an IL-12 gene sequence, for example, deletion or insertion of one or more bases, which do not affect the object of the present invention of minimized homologous recombination with a recombinant adenovirus vector. Therefore, the IL-12 gene sequence of the present invention is not limited to the sequence of SEQ ID NO: 1 or SEQ ID NO: 2 shown above, and all the examples of the IL-12 gene sequences should be interpreted to be included in the scope of the present invention as long as the sequences do not substantially affect the activity of the final product desired in the present invention.

In the present invention, small hairpin RNA or short hairpin RNA (shRNA) is an artificial RNA molecule having a hairpin structure, and is used to inhibit target gene expression through RNA interference. The shRNA that is comprised in the recombinant adenovirus of the present invention is complementary to VEGF mRNA, and suppresses VEGF gene expression. shRNA is usually delivered into cells via a plasmid, bacterial or virus vector. shRNA has an advantage of relatively low degradation and turnover ratios.

The term "shRNA molecule" used herein refers to a nucleic acid molecule that can mediate RNA interference or gene silencing. The shVEGF molecule used in the present invention includes a sequence complementary to VEGF mRNA, and the term "complementarity" used herein encompasses 100% complementarity, and a degree of incomplete complementarity sufficient to inhibit VEGF gene expression through an RNA interference mechanism, for example, preferably 90% complementarity, more preferably, 98% complementarity, and most preferably, 100% complementarity. In the specification, to represent 100% complementarity, it is specifically described as "completely complementary."

According to an exemplary embodiment of the present invention, the shRNA sequence included in the adenovirus of the present invention includes the sequence of SEQ ID NO: 3 (GenBank accession number gi: 70608153) or a sequence complementary to a part thereof, and preferably, a sequence complementary to the sequence of nucleotides 92 to 112 of an VEGF-A mRNA sequence, represented by the sequence of SEQ ID NO: 3. According to a specific exemplary embodiment of the present invention, the shRNA sequence is a sequence of SEQ ID NO: 4 and a sequence of SEQ ID NO: 5. Meanwhile, the sequence of SEQ ID NO: 3 has 88% homology with a sequence of SEQ ID NO: 6, and more specifically, 88% homology with the sequence of nucleotides 17 to 438 of the sequence of SEQ ID NO: 6.

Meanwhile, the recombinant adenovirus of the present invention includes an active E1A gene and a non-activated E1B 19 gene, an E1B 55 gene or an E1B 19/E1B 55 gene. In the specification, the term "non-activated" used in relation to a gene means that a normal protein encoded by the gene is not properly functioning since gene transcription and/or translation are/is not normally performed. For example, the non-activated E1B 19 gene is a gene that does not produce an active E1B 19 kDa protein due to a mutation (substitution, addition, partial deletion or entire deletion) occurring in the gene. E1B 19 deletion may result in an increase in necrosis ability, and the deletion of the E1B 55 gene results in tumor cell specificity (refer to Patent Application No. 2002-23760). The term "deletion" used in relation to a viral genomic sequence in the specification refers to partial deletion as well as complete deletion of a corresponding sequence.

According to an exemplary embodiment of the present invention, the recombinant adenovirus includes an E1A region, and E1B regions, containing E1B 19 and 55 kDa (ΔE1B), are deleted, and an E3 region (ΔE3) is deleted. The recombinant adenovirus including the E1A gene has a replicable characteristic. The IL-12 gene and shVEGF are inserted into the E1 and E3 region-deleted adenovirus, respectively (refer to FIG. 1A). However, the E1A region has a variation in which residue 45, Glu, is substituted with Gly in a nucleotide sequence encoding an Rb binding site located in the E1A gene sequence and a variation in which the sequence of amino acids 121 to 127 is entirely substituted with Gly.

The recombinant adenovirus used in the present invention includes a promoter operable in animal cells, preferably, mammalian cells. The proper promoter for the present invention includes a promoter derived from a mammalian virus and a promoter derived from the genome of mammalian cells, for example, a cytomegalovirus (CMV) promoter, an U6 promoter, a H1 promoter, a murine leukemia virus (MLV) long terminal repeat (LTR) promoter, an adenovirus early promoter, an adenovirus late promoter, a vaccina virus 7.5K promoter, an SV40 promoter, a HSV *tk* promoter, an RSV promoter, an EF1 α promoter, a methallothionin promoter, a β-actin promoter, a human IL-2 gene promoter, a human IFN gene promoter, a human IL-4 gene promoter, a human lymphotoxin gene promoter, a human GM-CSF gene promoter, a human phosphoglycerate kinase (PGK) promoter, a mouse phosphoglycerate kinase (PGK) promoter and a survivin promoter, but the present invention is not limited thereto. Most preferably, the promoter in the present invention is a CMV promoter.

In the recombinant adenovirus used in the present invention, the IL-12 gene sequence and the shVEGF sequence are operably linked to a promoter. In the specification, the term "operably linked" refers to a functional binding between a nucleic acid expression-regulatory sequence (e.g., an array of a promoter, a signal sequence, and a transcription regulating factor-binding site) and a different nucleic acid sequence, and therefore, the regulatory sequence regulates the transcription and/or translation of the different nucleic acid sequence.

The recombinant adenovirus of the present invention may further include an antibiotic-resistant gene and a reporter gene (e.g., green fluorescence protein (GFP), luciferase and β-glucuronidase) as selective markers. The antibiotic-resistant gene includes an antibiotic-resistant gene conventionally used in the art, for example, a gene resistant to ampicillin, gentamycin, carbenicillin, chloramphenicol, streptomycin, kanamycin, geneticin, neomycin and tetracycline, and preferably, is a neomycin-resistant gene. The selective marker may be expressed even in an expression system connected by a separate promoter or an internal ribosome entry site (IRES), and the IRES that can be used in the present invention is a regulatory sequence that is found in RNAs of some types of viruses and cells (McBratney et. al. Current Opinion in Cell Biology 5:961(1993)).

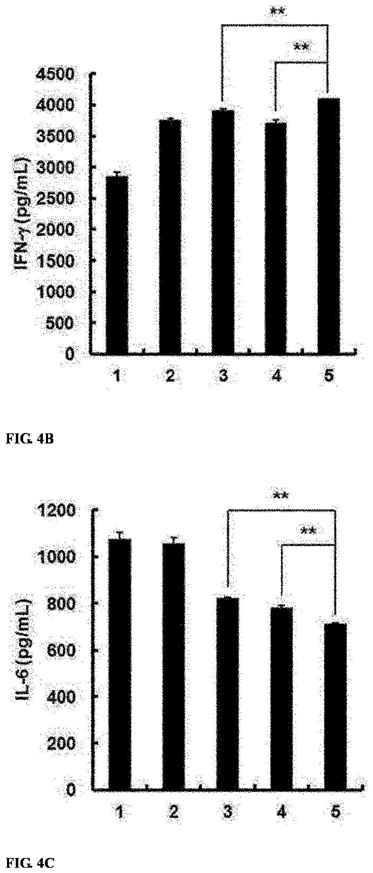

As to be described in an example described below, a recombinant adenovirus of the present invention, which co-expresses IL-12 and VEGF-specific shRNA, interrupts an immune surveillance avoiding mechanism of tumors by increasing IL-12 expression and suppressing VEGF expression. It has been reported that the shift from Th1 to Th2 cytokine expression has been shown in the development of various mouse and human malignant tumors, and the composition of the present invention increases a Th1/Th2 cytokine ratio by inhibiting such a Th2 immuno-deviation phenomenon (refer to FIGS. 4A to 4C).

In another aspect of the present invention, the present invention provides an anticancer composition which includes (a) a therapeutically effective amount of a recombinant adenovirus comprising (i) an interleukin (IL-12) gene having at least one of an IL-12A (p35) gene sequence and an IL-12B (p40) gene sequence, and (ii) a small hairpin RNA (shRNA) gene which is complementary to VEGF mRNA, and suppresses VEGF gene expression; and (b) a pharmaceutically acceptable carrier.

The anticancer composition of the present invention uses a recombinant adenovirus included in the above-described composition for enhancing antitumor immunity, and common details between these will be omitted to avoid excessive complexity in the specification.

According to an exemplary embodiment of the present invention, the cancer includes gastric cancer, lung cancer, breast cancer, ovarian cancer, liver cancer, bronchial cancer, nasopharyngeal cancer, laryngeal cancer, pancreatic cancer, bladder cancer, colorectal cancer, colon cancer, cervical cancer, brain cancer (e.g., glioma), prostate cancer, bone cancer, head and neck cancer, skin cancer (e.g., melanoma), kidney cancer, polyploid carcinoma, thyroid cancer, parathyroid cancer and ureteral cancer, but the present invention is not limited thereto.

In still another aspect of the present invention, the present invention provides a composition for use in a method of preventing or treating tumor-induced thymic atrophy, which includes (a) a therapeutically effective amount of a recombinant adenovirus comprising (i) an interleukin (IL-12) gene having at least one of an IL-12A (p35) gene sequence and an IL-12B (p40) gene, and (ii) a small hairpin RNA (shRNA) gene which is complementary to VEGF mRNA, and suppresses VEGF gene expression; and (b) a pharmaceutically acceptable carrier.

The composition for use in a method of preventing or treating tumor-induced thymic atrophy uses a recombinant adenovirus including in the above-described composition for enhancing antitumor immunity, and common details between these will be omitted to avoid excessive complexity in the specification.

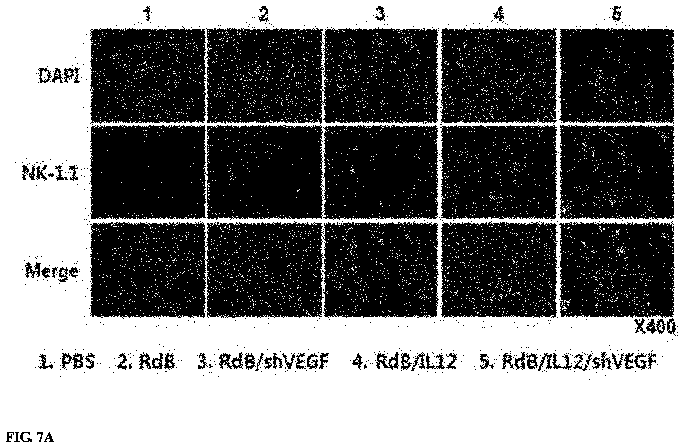

Thymic atrophy has been usually observed in tumor-bearing mice, and results in a reduction in host immunity with respect to tumors. The composition of the present invention co-expresses IL-12 and shVEGF in tumors, thereby preventing or inhibiting thymic atrophy caused by tumor development (refer to FIGS. 7A to 7G).

All of the compositions of the present invention described above include a pharmaceutically acceptable carrier. The pharmaceutically acceptable carrier used herein, conventionally used in formulation, includes lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate and mineral oil, but the present invention is not limited thereto. The pharmaceutical composition of the present invention may further include a lubricant, a wetting agent, a sweetening agent, a flavoring agent, an emulsifier, a suspension, a preservative, etc., other than the above-mentioned components. Suitable pharmaceutically acceptable carriers and agents are described in Remington's Pharmaceutical Sciences (19th ed., 1995) in detail.

The composition for use in a method of the present invention may be administered orally or parenterally, for example, by intravenously injection, subcutaneous injection, intramuscular injection, intraperitoneal injection or transdermal administration, and the composition of the present invention is preferably administered parenterally. According to an exemplary embodiment of the present invention, preferably, the composition for enhancing antitumor immunity of the present invention is directly intratumorally administered.

A suitable dosage of the pharmaceutical composition may be prescribed in various ways according to parameters such as a formation method, an administration method, a patient's age, body weight and sex, the severity of a disease symptom, a diet, administration time, administration route, excretion speed and reaction sensitivity. A daily dose of the pharmaceutical composition of the present invention is, for example, 0.001 to 1000 mg/kg. However, an actual dosage of the active ingredient may be determined by considering various related factors such as an amount of target tissue cells to be differentiated and proliferated, an administration route, a patient's weight, age and sex, and therefore, the dosage does not limit the scope of the present invention in any way.

### [Advantageous Effects]

Characteristics and advantages of the present invention are summarized as follows:
(a) The present invention relates to an oncolytic adenovirus co-expressing IL-12 and shVEGF as defined in claim 1, and a composition for use in a method of enhancing antitumor immunity and a composition for use in a method of enhancing an anticancer effect as defined in claim 5, which include the same.
(b) The inventors found that, when VEGF suppression and IL-12 expression are co-expressed in immunocompetent mouse melanoma models, immune functions are restored and an anticancer effect is enhanced.
(c) Particularly, the inventors identified that such an increase in anticancer effect is associated with an increase in anticancer immunity, an increase in Th1 cytokines and prevention of tumor-induced thymic atrophy, and thus first identified the applicability of a gene delivery system co-expressing IL-12 and shVEGF in cancer gene therapy.

### [Description of Drawings]

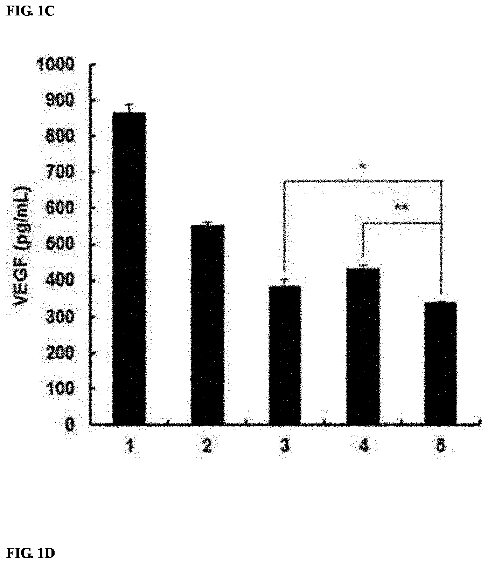

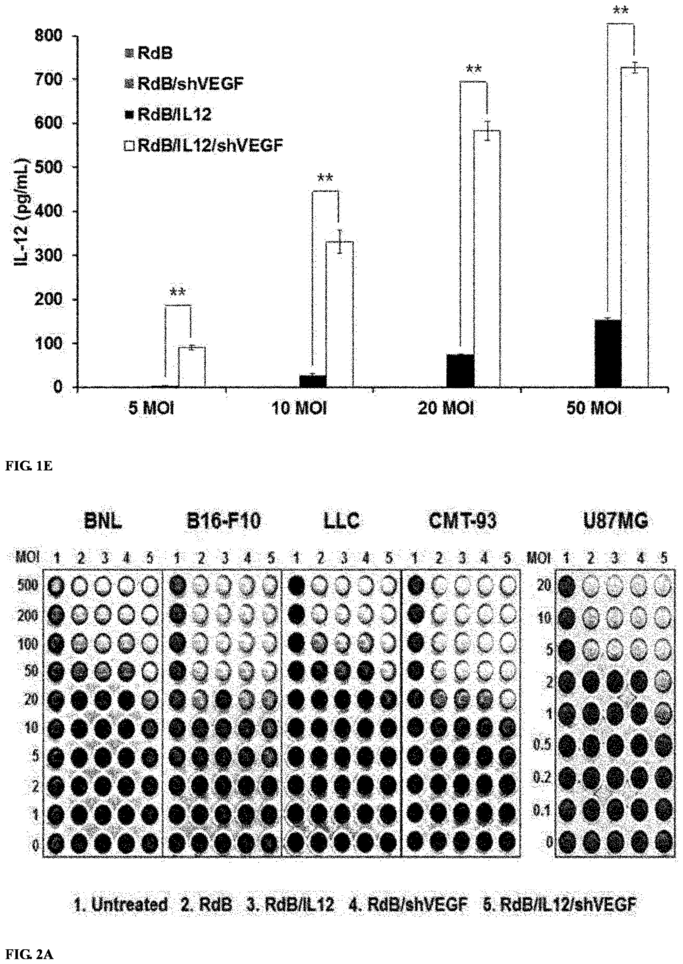

FIGS. 1A to 1E show the characteristics of oncolytic adenoviruses co-expressing IL-12 and VEGF shRNA. FIG. 1A shows schematic diagrams of genomic structures of adenovirus RdB and RdB/IL12/shVEGF. RdB includes mutated E1A (open star - a mutant of Rb protein-binding site), E1B 19, 55 kDa (ΔE1B) and E3 (ΔE3) regions are deleted; murine IL-12 and murine shVEGF were inserted into the E1 and E3 regions of the adenovirus genome. B16-F10 cells were infected with RdB, RdB/IL12, RdB/shVEGF or RdB/IL12/shVEGF, and IL-12 (FIG. 1B) and VEGF (FIG. 1C) expression levels were detected. Forty eight hours after infection, a cell culture supernatant was collected, and IL-12 and VEGF levels were quantified using an ELISA kit. Referring to FIG. 1D, by the same method as described above, IL-12 levels according to MOIs (5, 10, 20, 50 MOI) were quantified. FIG. 1E represents the cytopathic effects of the oncolytic adenovirus expressing IL-12 and/or shVEGF in murine cancer cell lines (BNL, B16-F10, LLC and CMT-93) and a human cancer cell line (U87MG). Data of triplicate experiments is expressed as the mean ± standard deviation (SD), and similar results were obtained from at least three independent experiments. MOI, multiplicity of infection. 1. PBS 2. RdB 3. RdB/IL12 4. RdB/shVEGF 5. RdB/IL12/shVEGF.

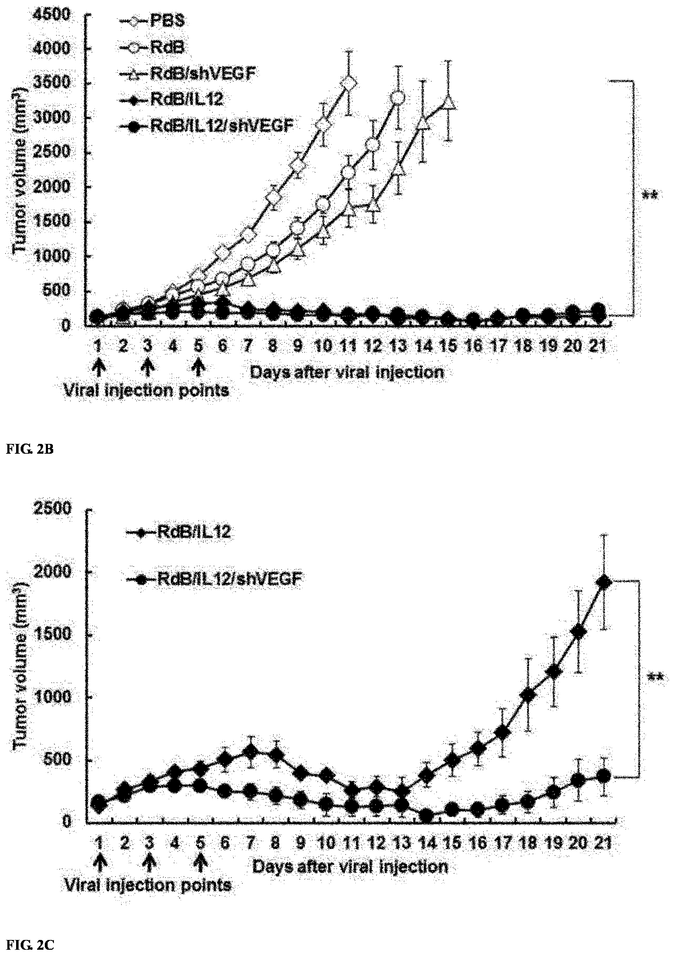

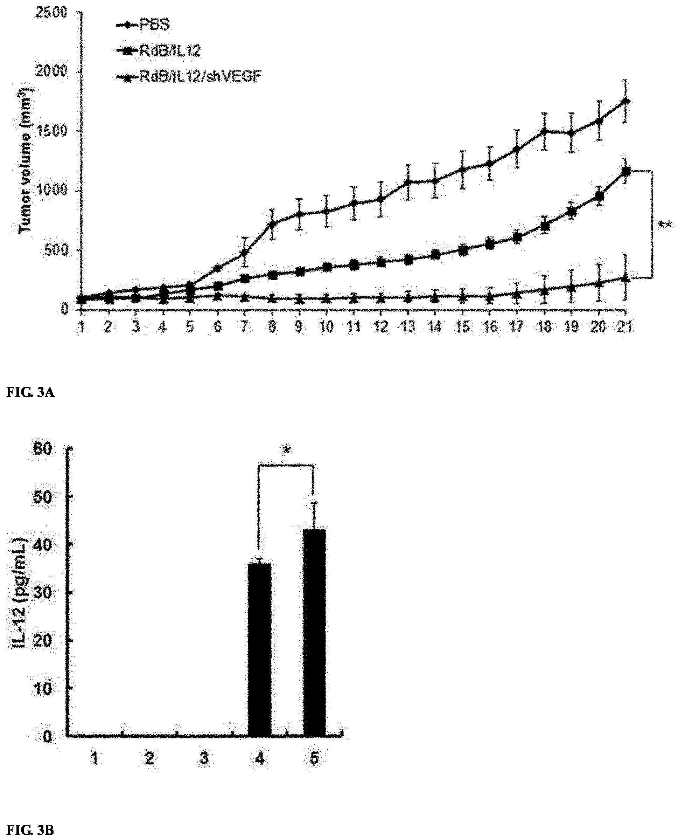

FIGS. 2A to 2C show the anticancer effects of adenoviruses in cancer-bearing mice. FIGS. 2A and 2B show the anticancer effects in melanoma mice treated with PBS (open diamonds), RdB (open circles), RdB/shVEGF (open triangles), RdB/IL12 (filled diamonds) or RdB/IL12/shVEGF (filled circles), and FIG. 2A to 2C show the anticancer effect in kidney cancer mice treated with PBS (open diamonds), RdB/IL12 (filled diamonds) or RdB/IL12/shVEGF (filled circles). Adenoviruses at a dose of 3 × 10⁹ VP (FIG. 2A; initial tumor volume: 80-100 mm³) or 6 × 10⁸ VP; and RdB/IL12 and RdB/IL12/shVEGF (FIG. 2B; initial tumor volume: 80-100 mm³, FIG. 2C; initial tumor volume: 100 mm³) adenoviruses were administered into tumors of C57BL/6 mice on day 1, day 3 and day 5. Tumor volume was monitored and recorded every day until the end of the study. Arrows represent injection time of adenoviruses. Values is expressed as the mean ± SD (n=8). **, P<0.01.

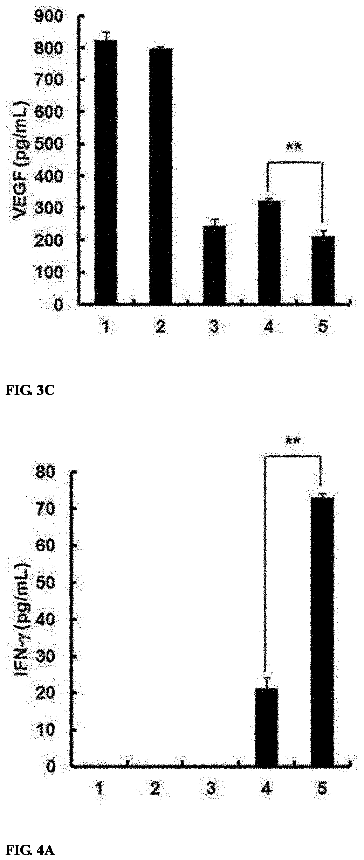

FIGS. 3A to 3C show in vivo intratumoral expression levels of IL-12, VEGF and IFN-γ. Seven days after initial viral injection, tumor tissues were collected, and IL-12 (FIG. 3A), VEGF (FIG. 3B) and IFN-γ (FIG. 3C) levels were measured by ELISA. Experiments were repeated three times, and data is expressed as the mean ± SD. *, P<0.05 or **, P<0.01; 1. PBS 2. RdB 3. RdB/shVEGF 4. RdB/IL12 5. RdB/IL12/shVEGF.

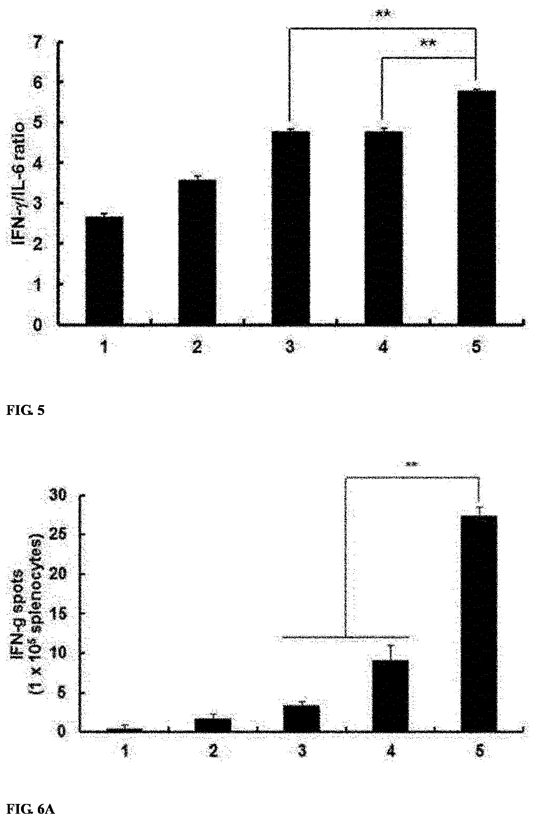

FIGS. 4A to 4C show that Th1/Th2 cytokine ratios were increased in splenocyte and B16-F10 co-cultured supernatants. On day 7 after the final viral injection, splenocytes were collected and cultured for 3 days with irradiated B16-F10 cancer cells in the presence of recombinant human IL-2. Th1/Th2/Th17 CBA was analyzed to measure the Th1/Th2 cytokine ratios of the supernatants. (FIG. 4A) IFN-γ value, (FIG. 4B) IL-6 value (FIG. 4C) IFN-y/IL-6 ratios. After triplicate experiments, data is expressed as the mean ± SD. ** P<0.01; 1. PBS 2. RdB 3. RdB/shVEGF 4. RdB/IL12 5. RdB/IL12/shVEGF.
FIG. 5 shows the result of measuring tumor-specific immunity. On day 7 after the final viral injection, splenocytes were collected from mice treated with PBS-, RdB- or cytokine-expressing adenoviruses, and cultured for 3 days with irradiated B16-F10 cells. Subsequently, the IFN-γ ELISPOT assay was carried out, and the number of spots was measured at a concentration of 1 × 10⁵ IFN-γ ELISPOT. After triplicate experiments, each value is expressed as the mean spot number ± SD. **, P<0.01; 1. PBS 2. RdB 3. RdB/shVEGF 4. RdB/IL12 5. RdB/IL12/shVEGF.

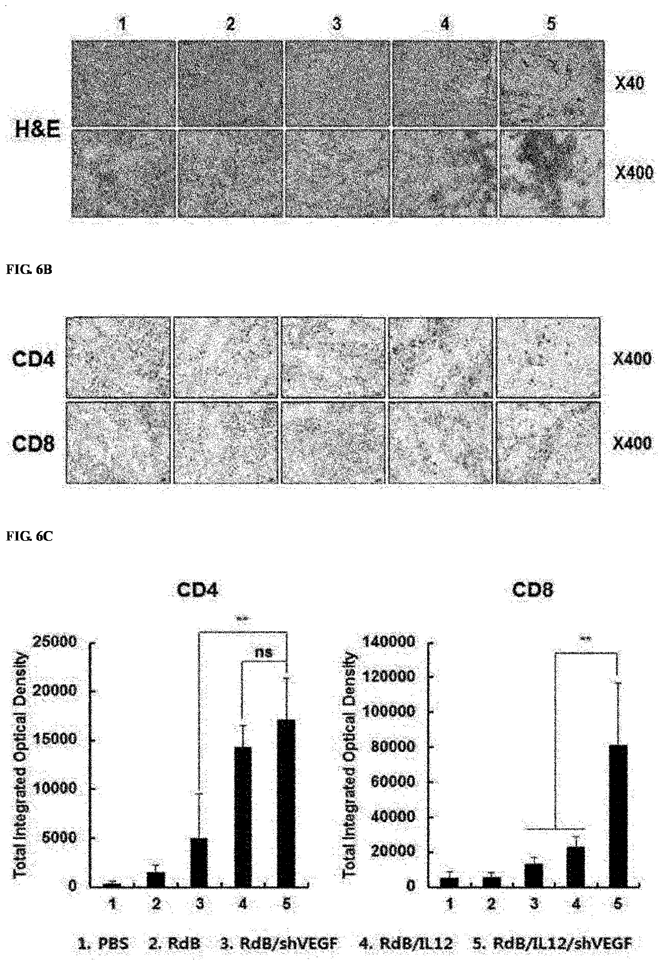

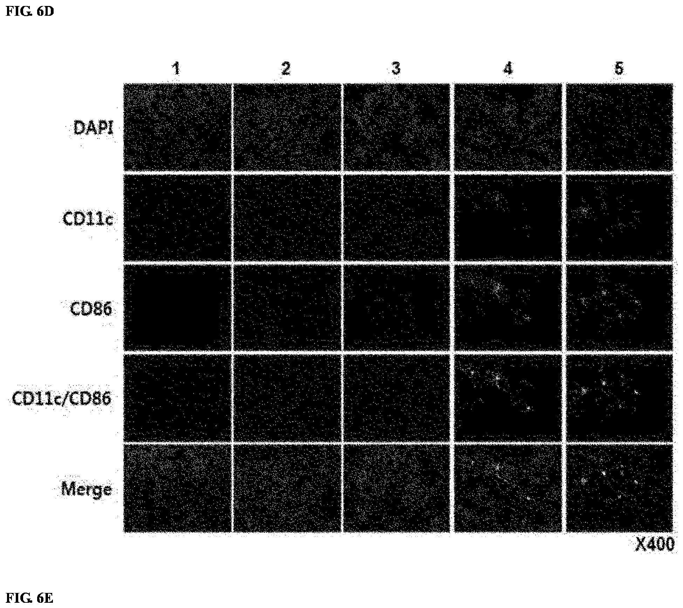

FIGS. 6A to 6E show the results of histological and immunohistochemical analyses for murine tumor fragments treated with adenoviruses. Adenoviruses were treated on days 1, 3 and 5, and tumors were collected on day 12, followed by histological analysis. FIG. 6A shows cryosections of tumor tissue were subjected to H&E staining. Magnification: 40× and 400×. FIG. 6B shows that cryosections of tumor tissue were stained with an anti-CD4 or anti-CD8 monoclonal antibody. Magnification: 400×. FIG. 6C shows increases in CD4+ T and CD8+ T expression that were semi-quantitatively measured using MetaMorph® image analysis software (**p<0.01). FIG. 6D shows cryosections of tumor tissue that were stained with anti-CDllc, anti-CD86 monoclonal antibodies, and DAPI. Magnification: 400×. FIG. 6E shows cryosections of tumor tissue that were stained with an anti-NK1.1 monoclonal antibody and DAPI. Magnification: 400×. 1. PBS 2. RdB 3. RdB/shVEGF 4. RdB/IL12 5. RdB/IL12/shVEGF.

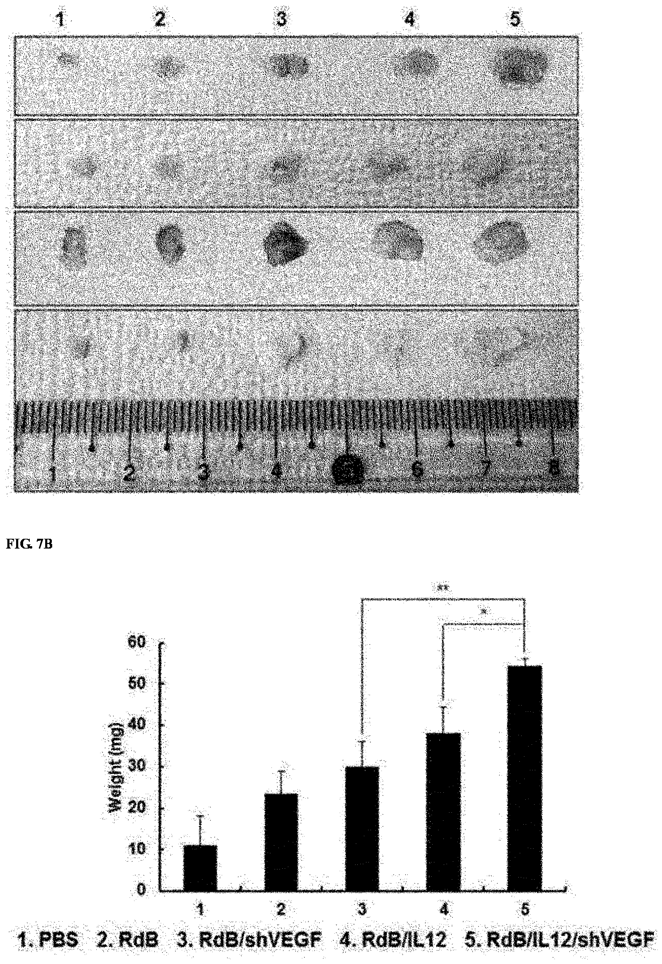

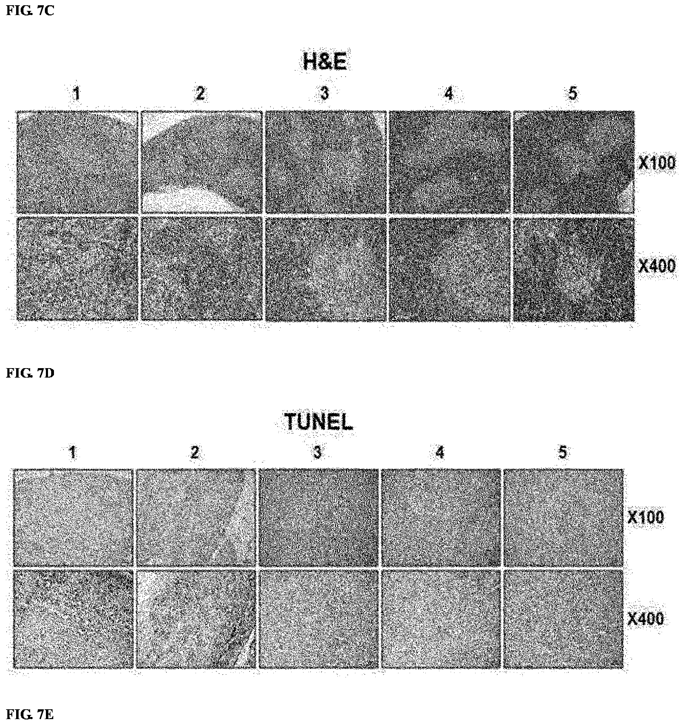

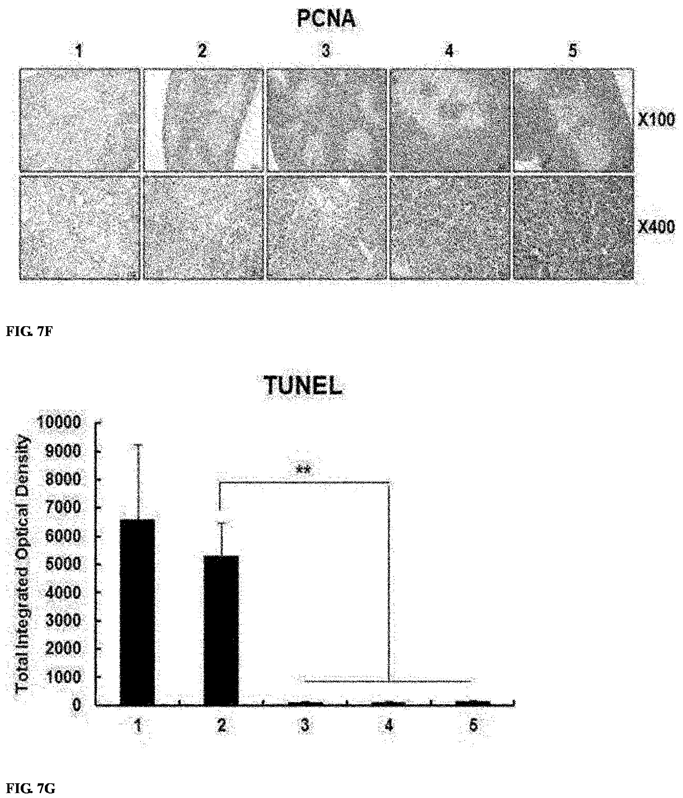

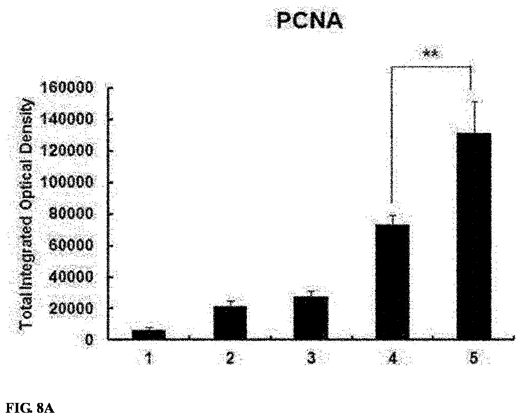

FIGS. 7A to 7G show the effect of preventing thymic atrophy of the RdB/IL12/shVEGF adenovirus. FIG. 7A shows images of the entire thymus of mice treated with PBS, RdB, RdB/IL12, RdB/shVEGF or RdB/IL12/shVEGF. FIG. 7B shows the weight of thymuses in mice treated with oncolytic adenoviruses. Data is expressed as the mean ± SD (* P<0.05, ** P<0.01). FIG. 7C shows sections from paraffin blocks that were stained with H&E. FIG. 7D shows the apoptosis in thymus tissue that was detected by TUNEL assay. FIG. 7E. shows the degree of proliferation of thymocytes as evaluated by proliferating cell nuclear antigen staining. FIGS. 7F and 7G show the results of FIGS. 7D and 7E that were semi-quantitatively measured using MetaMorph® image analysis software (**p<0.01). 1. PBS 2. RdB 3. RdB/shVEGF 4. RdB/IL12 5. RdB/IL12/shVEGF.

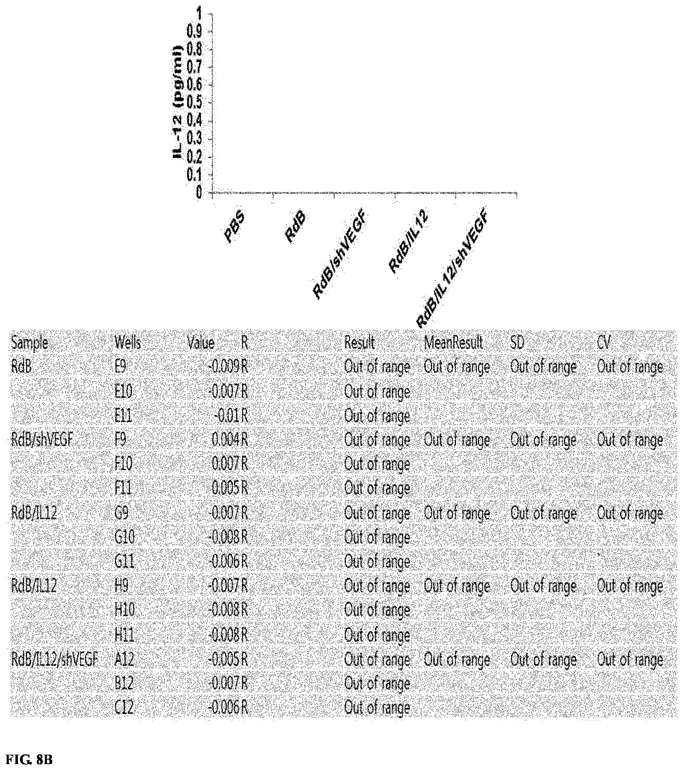

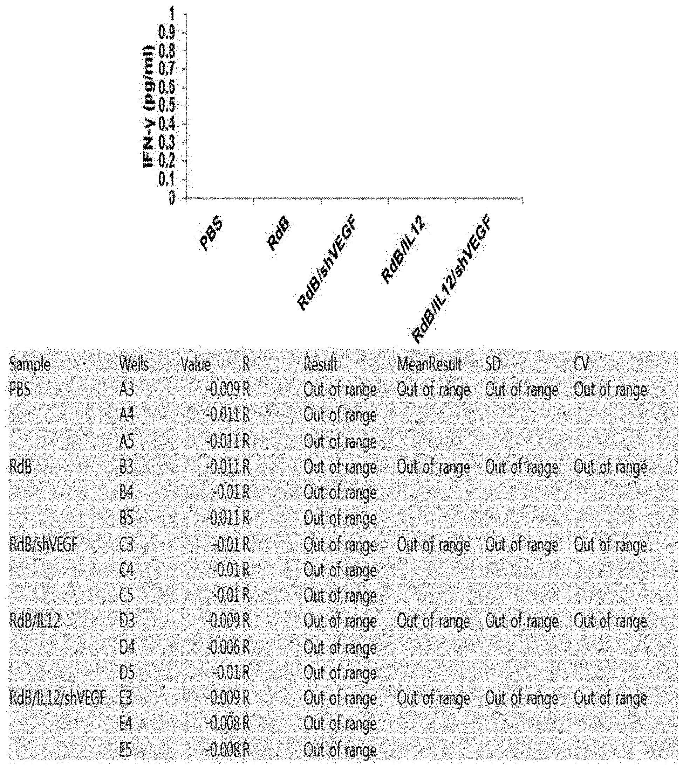

FIGS. 8A and 8B show the results obtained by ELISA assay following isolation of murine sera to confirm contents of IL-12 and IFN-γ remaining in mice on day 7 after final treatment of the mice with recombinant adenoviruses. FIG. 7A shows IL-12 contents in murine serum treated with PBS, RdB, RdB/shVEGF, RdB/IL12 or RdB/IL12/shVEGF. FIG. 7B shows IFN-γ contents in murine serum treated with PBS, RdB, RdB/shVEGF, RdB/IL12, or RdB/IL12/shVEGF.

### [Modes of the Invention]

Hereinafter, the present invention will be described in further detail with respect to examples.

### [Examples]

### Experimental materials and experimental methods

### (1) Cell lines and cell culture

Dulbecco's modified Eagle's medium (DMEM; Gibco BRL, Grand Island, NY) or Roswell Park Memorial Institute medium (RPMI; Gibco BRL), supplemented with 10% fetal bovine serum (FBS; Gibco BRL), L-glutamine (2 mmol/L), penicillin (100 IU/mL), and streptomycin (50 mg/mL), was used as a cell culture medium. HEK293 (human embryonic kidney cell line expressing an adenovirus E1 region), U87MG (human glioma cell line), BNL (murine liver cancer cell line), B16-F10 (murine melanoma cell line), LLC (murine lung cancer cell line), CMT-93 (murine polyploid carcinoma cell line), and Renca (murine renal adenocarcinoma cell line) were purchased from the American Type Culture Collection (ATCC; Manassas, VA). All cell lines were cultured at 37°C in a 5% CO₂ humid environment, and subjected to a mycoplasma negative test using Hoeschst dye, cell culture and polymerase chain reaction (PCR). Escherichia coli was cultured at 37°C in Luria Bertani medium.

### (2) Animal tests

Six- to eight-week-old male C57BL/6 mice were purchased from Charles River Laboratories International, Inc. (Wilmington, MA), and maintained in a laminar air-flow cabinet under a pathogen-free condition. All tests were approved by the Association for Assessment and Accreditation of Laboratory Animal Care (AAALAC), and conducted according to the guidelines established by the Hanyang University Institutional Animal Care and Use Committee.

### (3) Manufacture of oncolytic adenovirus co-expressing IL-12 and shVEGF

To manufacture adenoviruses co-expressing IL-12 and shVEGF in E1 and E3 regions, first, a shVEGF-expressing E3 shuttle vector was constructed. Full-length murine shVEGF complementary DNA was cloned by RT-PCR using total RNA obtained from bone marrow-derived active dendritic cells.

shVEGF complementary DNA (nucleotides 53-982 of National Center for Biotechnology Information L15435) was manufactured using the following primer set: sense primer (5'-gatcccggaaggagagcagaagtcccatgttcaagagacatgggacttctgctctcctt tttttttggaaa-3'), antisense primer (5'-tttccaaaaaaa aaggagagcagaagtcccatgtctcttgaacatgggacttctgctctccttccgggatc-3'). The PCR product was digested with BamHI/HindIII, and cloned in a BamHI/HindIII-treated pSP72 E3/CMV-poly A adenovirus E3 shuttle vector [29], thereby manufacturing a pSP72 E3/shVEGF E3 shuttle vector. For homologous recombination, the pSP72 E3/shVEGF was co-transfected with an adenovirus total vector pdE1 into *Escherichia coli* BJ5183, thereby manufacturing a pdE1/shVEGF adenovirus plasmid. The structure of the recombinant vector was confirmed by treatment of restriction enzymes and PCR analysis.

To construct an adenovirus E1 shuttle vector expressing IL-12, a murine IL-12 gene was cut out from pCA14/IL12 [30] and subcloned in a pXC1RdB E1 shuttle vector [23], thereby manufacturing a pXC1RdB/IL12E1 shuttle vector. For homologous recombination, the pXC1RdB/IL12 E1 shuttle vector and pdE1/shVEGF were co-transfected into *Escherichia coli* BJ5183, thereby manufacturing a pRdB/IL12/shVEGF adenovirus vector (FIG. 1A).

All viruses were produced using HEK293 cells, and purification, titration and quality analysis of the adenoviruses were carried out as described in the prior art [31, 32].

### (4) Enzyme-linked immunosorbent assay for IL-12 and VEGF expression

After 3 × 10⁵ B16-F10 melanoma cells were inoculated into a 6-well plate, and infected with 50 MOI of RdB, RdB/IL12, RdB/shVEGF, or RdB/IL12/shVEGF adenoviruses. Forty eight hours after the infection, a supernatant was collected, and subjected to measurement of IL-12 and shVEGF expression levels using an ELISA kit (IL-12 ELISA kit: Endogen, Woburn, MA; VEGF ELISA kit: R&D Systems, Minneapolis, MN).

### (5) Cytopathic effect assay

Cytopathic effect values are associated with a degree of virus replication, and able to be used to measure whether IL-12 and shVEGF expression affects the replicability of adenoviruses. Cells were inoculated into a 24-well plate to reach 30 to 80% confluence, and infected with 1-500 MOI of RdB, RdB/IL12, RdB/shVEGF or RdB/IL12/shVEGF adenoviruses. An apoptotic effect of viruses was visually monitored using a microscope. When virus-infected cells were completely dissolved at low MOI, dead cells were removed, and stained with 0.5% crystal violet in 50% methanol.

### (6) In vivo antitumor effect

B16-F10 cells (5 x 10⁵) or RENCA cells were injected subcutaneously into the right abdomen of 6- to 7-week-old male C57BL/6 mice. When the tumor volume reached approximately 100 mm³, the mice were divided into groups with similar tumor sizes, different doses of a total volume of 50 µl PBS-diluted adenoviruses (RdB or RdB/shVEGF at 3 × 10⁹ VP; RdB/IL-12 or RdB/IL-12/shVEGF at 6 × 10⁸ VP) were administered intratumorally three times every other day. Tumor growth was monitored everyday by measuring a perpendicular tumor diameter using a caliper. Tumor volume was calculated using the following formula: volume = 0.523L(W)², in which L is a length and W is a width.

### (7) Cytokine quantification and Th1/Th2/Th17 profiles in tumor tissue

Seven days after the final viral injection, tumor tissues were collected from an adenovirus-treated mouse group. The tumor tissues were homogenized in RIPA buffer (Elipis Biotech, Taejeon, South Korea) to which a proteinase inhibitor cocktail (Sigma, St. Louis, MO) was added. Following high speed centrifugation of the homogenate for 10 minutes, a total protein level was measured using a BSA ELISA kit (Pierce, Rockford, IL). Levels of IL-12, VEGF, and IFN-γ were measured by ELISA kits (IL-12 ELISA kit: Endogen; VEGF ELISA kit: R&D; IFN-γ ELISA kit: Endogen). Each experiment was conducted three times by group. Th1/Th2/Th17 type cytokine expression profiles in the tumor tissues were measured using a Th1/Th2/Th17 CBA kit (BD Biosciences Pharmingen, San Diego, CA). ELISA and CBA results were normalized with respect to a total protein concentration, and the results are shown as "pg/ total protein (mg)" values.

### (8) IFN-γ enzyme-linked immune spot (ELISPOT) assay in splenocytes

On day 7 after the final viral injection, spleens were collected aseptically from mice, and unicellular splenocytes were prepared [30]. The splenocytes were cultured with irradiated B16-F10 (5,000 rad) tumor cells for 3 days in the presence of recombinant human IL-2 (100 Uml-1; R&D Systems). Subsequently, IFN-γELISPOT assay was carried out [30]. Spots were measured using a computer-based immunospot system (AID Elispot Reader System, version 3.4; Autoimmun Diagnostika GmbH, Strassberg, Germany).

### (9) Histological and immunohistochemical analyses

Tumor tissues or thymus were fixed in 10% neutral buffered formalin, and a paraffin block was manufactured and then cut into 4-mm sections. The sections were stained with H&E, and observed using an optical microscope. To detect lymphocytes and dendritic cells, tumor tissues were frozen in an OCT compound (Sakura Finetec, Torrance, CA), and cut into 10-mm sections. The cryosections were reacted with rat anti-mouse CD4 monoclonal antibody (BD Biosciences Pharmingen), rat anti-mouse CD8 monoclonal antibody (BD Biosciences Pharmingen), mouse anti-mouse NK-1.1 monoclonal antibody (Biolegend, San Diego, CA), or rat anti-mouse CD86 monoclonal antibody (BD Biosciences Pharmingen) as a primary antibody, and reacted with horseradish peroxidase-conjugated goat anti-rat IgG (BD Biosciences Pharmingen) or horseradish peroxidase-conjugated goat anti-mouse IgG (Southern Biotech, Birmingham, AL) as a secondary antibody. Diaminobenzidine/hydrogen peroxidase (DAKO, Copenhagen, Denmark) was used as a chromogenic substrate. All sides were counterstained with Meyer's hematoxylin. To detect proliferated cells, the slide was deparaffinated with xylene, and dehydrated by treatment of an alcohol. Intrinsic peroxidase activity was inhibited using 3% hydrogen peroxide. An anti-proliferating cell nuclear antigen monoclonal antibody PC10 (DAKO) was bound to the secondary antibody Real/Envision (DAKO). A mouse serum was added to the complex of the first and second antibodies to minimize the interaction between isolated second antibodies and murine immunoglobulins found in the tissue sections. Subsequently, the sections were reacted with a complex of the first antibody and the second antibody, followed by the reaction with streptavidin-peroxidase. Diaminobenzidine/hydrogen peroxidase was used as a chromogenic substrate. Expression levels of CD4, CD8, NK-1.1, CD86, and PCNA were semi-quantitatively analyzed using MetaMorph® image analysis software (Universal Image Corp., Buckinghamshire, UK). Results were expressed as the mean optical density of five different digital images.

### (10) Extraction of thymuses

B16-F10 murine melanoma cells were suspended in a 100 ml Hank's balanced salt solution, and then injected into the abdomen of 6- to 8-week-old male C57BL/6 mice. When the volume of the tumor reached 80 to 100 mm³ (8-10 days after cell implantation), the mice were treated with adenoviruses (RdB or RdB/shVEGF at 1 × 10¹⁰ VP / tumor cell-suspended PBS at 50 µl; RdB/IL-12 or RdB/IL-12/shVEGF at 5 ×10⁹ VP / tumor cell-suspended PBS at 50 µl) every other day for three times. On day 7 after the final viral injection, all thymus tissues were extracted and immediately added to RPMI 1640 medium, and each thymus was weighed using an electronic scale (Ohaus Corp., Florham Park, NJ).

### (11) Terminal Deoxynucleotidyl Transferase dUTP Nick End Labeling (TUNEL) assay

An apoptotic thymocyte population was observed by TUNEL assay [33]. The apoptotic cells were visually identified in five randomly selected regions, and photographed at magnifications of 100× and 400×. The expression level of the apoptotic thymocytes was semi-quantitatively analyzed using MetaMorph® image analysis software. Results are expressed as the mean optical density of five different digital images.

### (12) Statistical analysis

All data is expressed as the mean ± SD. Statistical comparisons were made using Stat View software (Abacus Concepts, Inc., Berkeley, CA) and the Mann-Whitney test (non-parametric rank sum test). P values less than 0.05 were considered statistically significant (*, P<0.05; **, P<0.01).

### Experimental results

### (1) Oncolytic adenovirus-mediated IL-12 and shVEGF expression

To generate oncolytic adenoviruses effectively inhibiting the expression of long-term VEGF, an oncolytic adenovirus co-expressing IL-12 and shVEGF was manufactured by inserting murine IL-12 (p35, IRES, and p40) and shVEGF genes into the E1 and E3 regions of the adenovirus (RdB) [23] (FIG. 1A). In RdB/IL12/shVEGF, the level of IL-12 expression was measured, and to examine whether VEGF-specific shRNA inhibits VEGF expression, B16-F10 melanoma cells were infected with the adenoviruses at a multiplicity of infection (MOI) of 50 using RdB, RdB/IL12, RdB/shVEGF, or RdB/IL12/shVEGF. 48 hours after infection, the IL-12 or VEGF level in a supernatant was measured by ELISA. As shown in FIGS. 1B and 1C, the level of IL-12 secreted from cells infected with 50 MOI of RdB/IL12/shVEGF was 181.4 ± 10.0 pg/mL, but was hardly detected in RdB- or RdB/shVEGF-infected cells, and only detected at 35.6 ± 4.0 pg/mL in RdB/IL12-infected cells. In addition, the level of VEGF expression in RdB/IL12/shVEGF (MOI of 50)-infected cells was 338.0 ± 6.1 pg/mL, and 552.1 ± 10.3 pg/mL, 431.1 ± 11.2 pg/mL, and 384.6 ± 19.4 pg/mL in RdB-, RdB/IL12-, and RdB/shVEGF-infected cells, respectively. Such results, compared with RdB/IL12 or RdB/shVEGF, show that RdB/IL12/shVEGF was increased IL-12 secretion 5-fold, and decreased VEGF secretion 1.2-fold. In addition, as a result of measuring changes in IL-12 secretion according to MOIs (5, 10, 20, 50 MOIs), like the above result, a considerable increase in IL-12 secretion in RdB/IL12/shVEGF was dependent on the concentration of the viruses, but IL-12 secretion was hardly detected in RdB- or RdB/shVEGF-infected cells. In the case of RdB/IL12, IL-2 was detected, but there was a considerable difference when RdB/IL12/shVEGF was introduced (FIG. 1D).

To examine whether IL-12, shVEGF, or IL-12/shVEGF expression affects virus replication and oncolytic ability, in mouse cell lines (BNL, B16-F10, LLC, and CMT-93) with different histological types and a human cell line (U87MG), the ability to induce cytopathic effects of RdB/IL12, RdB/shVEGF, and RdB/IL12/shVEGF was measured. The human U87MG cell line exhibited higher sensitivity to adenovirus infection, than the mouse cells, and the human cell line was used for *in vitro* experiments. Cells were infected with RdB (oncolytic adenovirus species), RdB/IL12, RdB/shVEGF, or RdB/IL12/shVEGF, and then stained with crystal violet to observe cell lysis. As shown in FIG. IE, the cytopathic effects of RdB/IL12, RdB/shVEGF, or RdB/IL12/shVEGF were equal or higher than RdB, and the high expression of IL-12 and shVEGF did not reduce the replicability and oncolytic ability of adenoviruses.

### (2) Confirmation of anticancer effect in cancer-bearing mouse models

When compared with a single-use effect of RdB/IL-12 or RdB/shVEGF, to examine the therapeutic efficacy of RdB/IL12/shVEGF *in vivo,* first, B16-F10 melanoma in C57BL/6 mice were injected with RdB, RdB/IL12, RdB/shVEGF, or RdB/EL12/shVEGF. When the tumor volume averaged 80 to 100 mm³, virus treatment was started, and was performed total three times every other day. A PBS solution containing 3 × 10⁹ virus particles (VPs) was treated. Tumors in PBS control mice were rapidly grown to become huge tumors, and the average tumor volume until 5 days after the final treatment was 3,000 mm³ or more (FIG. 2A). Meanwhile, the RdB-, RdB/IL12-, RdB/shVEGF-, or RdB/IL12/shVEGF-treated group was considerably reduced in tumor volume, and the average tumor volume was 1747.4 ± 127.2 mm³, 207.7 ± 35.2 mm³, 1381.2 ± 194.7 mm³, or 157.3 ± 49.0 mm³, respectively. The tumor growth inhibition rates, compared with the PBS control, were 40% (RdB), 93% (RdB/IL12), 53% (RdB/shVEGF), and 95% (RdB/IL12/shVEGF). Five of 8 mice in RdB/IL12- and RdB/IL12/shVEGF-treated groups showed complete remission, whereas none of mice in other mouse groups (PBS, RdB, and RdB/shVEGF) showed complete remission. However, all IL-12-expessing adenoviruses (RdB/IL12 and RdB/IL12/shVEGF) showed considerable antitumor effects, whereas there was no considerable difference in the tumor inhibitory effect between RdB/IL12- and RdB/IL12/shVEGF-treated mouse groups. Therefore, the inventors measured the antitumor effects of RdB/IL12 and RdB/IL12/shVEGF at a 5-fold lower dose than the previous experiment using 6 × 10⁸ VP (FIG. 2B). On day 15 after the first administration of 6 × 10⁸ VP, tumors had regrown to a volume of 502.5 ± 128.9 mm³ in the RdB/IL12-treated group, whereas tumor growth was still inhibited and thus the tumor volume was 106.1 ± 52.9 mm³ in RdB/IL12/shVEGF treated groups, indicating that, compared with the RdB/IL12 group, there was an excellent tumor inhibitory effect (**, P<0.01). In addition, 6 × 10⁸ VP of RdB, RdB/IL12, RdB/IL12/shVEGF were introduced into RENCA kidney cancer cells of C57BL/6 mice, and their anticancer effects were examined. As described above, it can be seen that the PBS control mice showed the generation of huge tumors due to rapid growth of tumors on about day 7 or 8, and the RdB/IL12/shVEGF-related mouse group also showed a considerably excellent tumor growth inhibitory effect (FIG. 2C), which had a significant difference, when compared to RdB/IL12-treated group (**, P<0.01).

Such results infer that, in B16-F10 murine melanoma or RENCA kidney cancer models, RdB/IL12/shVEGF has a superior antitumor activity compared to the RdB/IL12 or RdB/shVEGF group.

### (3) Increased local expression of IL-12, VEGF, and IFN-γ in tumor tissues

To measure the levels of IL-12 and VEGF produced in RdB/IL12, RdB/shVEGF, or RdB/IL12/shVEGF-treated mice, tumor tissues were harvested 7 days after final viral injection. As shown in FIG. 3A, IL-12 expression was not observed in tumors of PBS-, RdB-, and RdB/shVEGF-treated groups. However, high concentrations of IL-12 were shown in tumors in RdB/IL12 or RdB/IL12/shVEGF-treated groups (36.0 ± 1.0 pg/mL and 43.0 ± 6.0 pg/mL, respectively; *, P<0.05). In addition, PBS, compared with the PBS-, RdB-, or RdB/IL12-treated group (823.2 ± 26.2, 796.3 ± 6.7, and 320.6 ± 8.0 pg/mL, respectively; **, P<0.01), tumors in the RdB/shVEGF- or RdB/IL12/shVEGF-treated group exhibited significantly lower VEGF levels (242.5 ± 22.5 pg/mL and 212.0 ± 15.7 pg/mL, respectively) (FIG. 3B). Such results showed that, compared with when using onlyan RdB/IL12 or RdB/shVEGF composition, in treatment using a mixed composition, IL-12 expression was further increased and VEGF expression was further decreased.

IL-12-induced IFN-γ attenuated VEGF levels *in vivo* [24]. Therefore, the levels of IFN-γ expression in tumor tissues were measured. As shown in FIG. 3C, the RdB/IL12- or RdB/IL12/shVEGF-treated group showed high IFN-γ levels, and particularly, the RdB/EL12/shVEGF-cotreated group showed considerably higher expression levels than the RdB/IL12 only-administered group (21.0 ± 3.0 pg/mL: RdB/IL12, 73.0 ± 1.0 pg/mL: RdB/IL12/shVEGF, **, P<0.01), whereas IFN-γ expression was not detected in the PBS-, RdB-, and RdB/shVEGF-treated groups. That is, tumors in the RdB/IL12/shVEGF-treated group showed considerably higher IL-12 and IFN-γ levels, and a lower VEGF level than other groups.

### (4) Increase in Th1/Th2 cytokine ratio

The shift from Th1 to Th2 cytokine expression has been reported to be shown in the growth of malignant tumors in various mice and a human, and a correlation between the cytokine level and cancer therapeutic efficacy has also been reported [25, 26]. Therefore, the inventors investigated whether RdB/IL12/shVEGF, which allows Th1 cytokine (e.g., IFN-γ) expression to be considerably increased, could shift a Th2 immune response to a Th1 immune response in splenocytes. Splenocytes of mice were harvested 7 days after final viral injection, and cultured with tumor cells in irradiated B16-F10 mice for 3 days in the presence of recombinant human IL-2. Afterward, the cultured supernatant was analyzed using a Th1/Th2/Th17 cytometric bead assay (CBA) kit. The Th1/Th2 cytokine ratio was calculated from the IFN-y/IL-6 ratio. As shown in FIGS. 4A to 4C, RdB/IL12/shVEGF-treated splenocytes of B16-F10 mice exhibited the highest IFN-y/IL-6 ratio among the PBS-, RdB-, RdB/IL12- and RdB/shVEGF-treated groups (**, P<0.01). IL-2, IL-4, IL-10, and IL-17 were not detected. Such results showed that RdB/EL12/shVEGF suppressed Th2 cytokines and increased Th1 cytokines, resulting in deviation of T cell responses in Type 1 pattern.

### (5) Occurrence of tumor-specific immune responses

Compared with oncolytic adenoviruses expressing one of IL-12 and shVEGF, RdB/IL12/shVEGF creates a tumor environment advantageous for inducing tumor-specific immune responses. Therefore, the inventors measured the number of immune cells expressing IFN-γ, which is the cytokine secreted from activated T cells, and an *in vivo* experiment to determine whether RdB/IL12/shVEGF increases antitumor immune responses was carried out. Splenocytes of mice were obtained 7 days after final viral injection, and cultured with tumor cells of irradiated B16-F10 mice for 24 hours in the presence of recombinant human IL-2. Afterward, IFN-γ-secreted immune cells were measured in splenocytes using an IFN-γ ELISPOT kit. As shown in FIG. 5, the frequency of IFN-γ-secreted immune cells in the RdB/IL12/shVEGF-injected mice was considerably higher than cells in the RdB/IL12- or RdB/shVEGF-treated mice. Particularly, the number of IFN-γ-secreted immune cells (27.3 ± 1.1) isolated from 1 × 10⁵ cells of the RdB/IL12/shVEGF-treated group was much higher than those of the PBS-, RdB-, RdB/IL12-, and RdB/IL12/shVEGF-treated groups (0.3 ± 0.5, 1.6 ± 0.5, 9.0 ± 2.0, and 3.3 ± 0.5, **, P<0.01). These results indicate that the RdB/IL12/shVEGF-treated group exhibited higher tumor-specific immune responses than the RdB/IL12 or RdB/shVEGF-treated group.

### (6) Increased infiltration of CD4+ T cells, CD8+ T cells and dendritic cells into RdB/IL12/shVEGF-treated tumors

To examine the histological characteristics of tumor tissues shown after RdB/IL12/shVEGF treatment, tumor tissues were stained with hematoxylin and eosin (H&E) for analysis. Histological analysis showed increased tumor necrosis in tumor tissues of the RdB/IL12/shVEGF-administered mice, compared with the RdB/IL12 and RdB/shVEGF-treated groups. However, almost all tumor cells died in the RdB/IL12/shVEGF-treated group. Immune cells infiltrated into the tumor tissues were identified by immunohistochemical analysis using CD4-, CD8-, CD11c, and CD86-specific antibodies. When compared with the RdB/IL12- or RdB/shVEGF-treated group, in the RdB/IL12/shVEGF-treated group, high frequencies of CD4+ T, CD8+ T, CD86, and CD11c were observed in tumors in the central and boundary regions (FIGS. 6A, 6B, 6D, and 6E). The increased CD4+ T and CD8+ T expression was semi-quantitatively measured using MetaMorph® image analysis software (FIG. 6C).

These results indicated that the co-expression of IL-12 and shVEGF in tumor tissues could induce strong activation and recruitment of T cells as well as dendritic cells. However, IL-12- and shVEGF-single expression are not effective for recruitment of T cells and dendritic cells to tumor tissues. As a result, the strongest anticancer immune responses are shown in tumor tissues due to intratumoral injection of adenoviruses co-expressing IL-12 and shVEGF.

### (7) Prevention of tumor-induced thymic atrophy by RdB/IL12/shVEGF treatment

Thymic atrophy is generally observed in tumor-bearing mice, and induces the suppression of host immunity against a tumor [27, 28]. In addition, even long-term exposure to recombinant VEGF induced thymic atrophy *in vivo* [21]. As a result, the prevention of thymic atrophy is important in overcoming tumor-induced immunosuppression. Therefore, the inventors measured changes in thymus volume and weight of adenovirus-treated mice. As seen from FIG. 7A, thymic atrophy was not observed in RdB/IL12/shVEGF-treated mice, whereas thymic atrophy was observed in PBS and RdB-treated mice, and on day 7 after final viral injection, the weight of thymuses of tumor-bearing mice was considerably reduced. However, in RdB/IL12- or RdB/shVEGF-treated mouse group, thymic atrophy became a little alleviated, and its effect was insignificant. However, the RdB/IL12/shVEGF-treated mouse group had much heavier thymus weights (FIG. 7B), which were inversely proportional to tumor volumes. Such results indicated that thymic atrophy was induced by tumor growth, and could be prevented by co-expressing IL-12 and shVEGF in tumors.

Subsequently, to examine a change in thymuses of oncolytic adenovirus-treated mice, histological and immunohistochemical staining was performed on thymic tissues. Normal morphology was observed in the thymic tissues, and this result showed that, on day 7 after final injection of RdB/IL12-, RdB/shVEGF-, and RdB/IL12/shVEGF adenoviruses, the cortex and medulla regions in mice were clearly separated (FIG. 7C). However, it could be seen that the thymic structure in mice was abnormally disrupted on day 7 after final injection of PBS or RdB. TUNEL analysis results showed that, compared with the RdB/IL12-, RdB/shVEGF-, or RdB/IL12/shVEGF-treated mice, abundant apoptosis was observed in thymic tissues in PBS- or RdB-treated mice (FIG. 7D). In addition, it was proven that the RdB/IL12/shVEGF-treated mice showed positive in proliferating cell nuclear antigen (PCNA) staining, indicating active proliferation of thymic cells in thymuses (FIG. 7E). The data were semi-quantitatively measured using MetaMorph® image analysis software (**p<0.01) (FIGS. 7F and 7G). These results indicated that RdB/IL12/shVEGF induces cell proliferation in thymuses and inhibits apoptosis, thereby preventing thymic atrophy in tumor-bearing mice.

### (8) Examination of systemic toxicity using animal models

To examine systemic toxicity caused by treatment of recombinant adenoviruses (PBS, RdB, RdB/shVEGF, RdB/IL12, or RdB/IL12/shVEGF), specifically, 7 days after final treatment of the recombinant adenoviruses, the contents of IL-12 and IFN-γ remaining in mouse serum were quantified using an ELISA kit. As a result, in all sera of the RdB/shVEGF-, RdB/IL12-, or RdB/IL12/shVEGF-treated mice, as well as PBS- or RdB-treated mice, neither IL-12 nor IFN-γ was detected at all, indicating that the recombinant adenoviruses according to the present invention did not cause systemic toxicity.

### References

1. Kavanaugh, D.Y. and D.P. Carbone, Immunologic dysfunction in cancer. Hematol Oncol Clin North Am, 1996. 10(4): p. 927-51.
2. Reuther, T., et al., Osteoradionecrosis of the jaws as a side effect of radiotherapy of head and neck tumor patients--a report of a thirty-year retrospective review. Int J Oral Maxillofac Surg, 2003. 32(3): p. 289-95.
3. Lindley, C., et al., Perception of chemotherapy side effects cancer versus noncancer patients. Cancer Pract, 1999. 7(2): p. 59-65.
4. KruC., T. Greten, and F. Korangy, Immune based therapies in cancer. 2007.
5.Gabrilovich, D.I., et al., Production of vascular endothelial growth factor by human tumors inhibits the functional maturation of dendritic cells. Nat Med, 1996. 2(10): p. 1096-103.
6. Dunn, G.P., L.J. Old, and R.D. Schreiber, The immunobiology of cancer immunosurveillance and immunoediting. Immunity, 2004. 21(2): p. 137-48.
7. Huang, B., et al., Toll-like receptors on tumor cells facilitate evasion of immune surveillance. Cancer Res, 2005. 65(12): p. 5009-14.
8. Chen, Q., et al., Production of IL-10 by melanoma cells: examination of its role in immunosuppression mediated by melanoma. Int J Cancer, 1994. 56(5): p. 755-60.
9. Ormandy, L.A., et al., Increased populations of regulatory T cells in peripheral blood of patients with hepatocellular carcinoma. Cancer Res, 2005. 65(6): p. 2457-64.
10. Zou, W., Regulatory T cells, tumor immunity and immunotherapy. Nat Rev Immunol, 2006. 6(4): p. 295-307.
11. Kusmartsev, S. and D.I. Gabrilovich, Role of immature myeloid cells in mechanisms of immune evasion in cancer. Cancer Immunol Immunother, 2006. 55(3): p. 237-45.
12. Aste-Amezaga, M., et al., Cooperation of natural killer cell stimulatory factor/interleukin-12 with other stimuli in the induction of cytokines and cytotoxic cell-associated molecules in human T and NK cells. Cell Immunol, 1994. 156(2): p. 480-92.
13. Trinchieri, G., Interleukin-12 and the regulation of innate resistance and adaptive immunity. Nat Rev Immunol, 2003. 3(2): p. 133-46.
14. Robertson, M.J., et al., Interleukin 12 immunotherapy after autologous stem cell transplantation for hematological malignancies. Clin Cancer Res, 2002. 8(11): p. 3383-93.
15. Younes, A., et al., Phase II clinical trial of interleukin-12 in patients with relapsed and refractory non-Hodgkin's lymphoma and Hodgkin's disease. Clin Cancer Res, 2004. 10(16): p. 5432-8.
16. Atkins, M.B., et al., Phase I evaluation of intravenous recombinant human interleukin 12 in patients with advanced malignancies. Clin Cancer Res, 1997. 3(3): p. 409-17.
17. Robertson, M.J., et al., Immunological effects of interleukin 12 administered by bolus intravenous injection to patients with cancer. Clin Cancer Res, 1999. 5(1): p. 9-16.
18. Haicheur, N., et al., Cytokines and soluble cytokine receptor induction after IL-12 administration in cancer patients. Clin Exp Immunol, 2000. 119(1): p. 28-37.
19. Portielje, J.E., et al., Repeated administrations of interleukin (IL)-12 are associated with persistently elevated plasma levels of IL-10 and declining IFN-gamma, tumor necrosis factor-alpha, IL-6, and IL-8 responses. Clin Cancer Res, 2003. 9(1): p. 76-83.
20. Neufeld, G., et al., Vascular endothelial growth factor (VEGF) and its receptors. Faseb j, 1999. 13(1): p. 9-22.
21. Ohm, J.E., et al., VEGF inhibits T-cell development and may contribute to tumor-induced immune suppression. Blood, 2003. 101(12): p. 4878-86.
22. Su, J.L., et al., The VEGF-C/Flt-4 axis promotes invasion and metastasis of cancer cells. Cancer Cell, 2006. 9(3): p. 209-23.
23. Kim, J., et al., E1A- and E1B-Double mutant replicating adenovirus elicits enhanced oncolytic and antitumor effects. Hum Gene Ther, 2007. 18(9): p. 773-86.
24. Dias, S., R. Boyd, and F. Balkwill, IL-12 regulates VEGF and MMPs in a murine breast cancer model. Int J Cancer, 1998. 78(3): p. 361-5.
25. Smyth, M.J., et al., Cytokines in cancer immunity and immunotherapy. Immunol Rev, 2004. 202: p. 275-93.
26. Gadducci, A., et al., Serum tumor markers in the management of ovarian, endometrial and cervical cancer. Biomed Pharmacother, 2004. 58(1): p. 24-38.
27. Carrio, R. and D.M. Lopez, Impaired thymopoiesis occurring during the thymic involution of tumor-bearing mice is associated with a down-regulation of the antiapoptotic proteins Bcl-XL and A1. Int J Mol Med, 2009. 23(1): p. 89-98.
28. Fu, Y., et al., Thymic involution and thymocyte phenotypic alterations induced by murine mammary adenocarcinomas. J Immunol, 1989. 143(12): p. 4300-7.
29. Yun, C.O., et al., ADP-overexpressing adenovirus elicits enhanced cytopathic effect by induction of apoptosis. Cancer Gene Ther, 2005. 12(1): p. 61-71.
30. Lee, Y.S., et al., Enhanced antitumor effect of oncolytic adenovirus expressing interleukin-12 and B7-1 in an immunocompetent murine model. Clin Cancer Res, 2006. 12(19): p. 5859-68.
31. Zhang, S.N., et al., Optimizing DC vaccination by combination with oncolytic adenovirus co-expressing IL-12 and GM-CSF. Mol Ther, 2011. 19(8): p. 1558-68.
32. Choi, K.J., et al., Strengthening of antitumor immune memory and prevention of thymic atrophy mediated by adenovirus expressing IL-12 and GM-CSF. Gene Ther, 2012. 19(7): p. 711-23.
33. Yoo, J.Y., et al., Short hairpin RNA-expressing oncolytic adenovirus-mediated inhibition of IL-8: effects on antiangiogenesis and tumor growth inhibition. Gene Ther, 2008. 15(9): p. 635-51.
34. Khan W I et al., IL-12 gene transfer alters gut physiology and host immunity in nematode-infected mice. American Journal of Physiology - Gastrointestinal and liver physiology, American Physiological Society, US; vol. 281 no. 1, 30 June 2001, pages G102-G110.
35. Liang Wen et al., In vitro induction of specific anti-tumoral immunity against laryngeal carcinoma by using human interleukin-12 gene-transfected dendritic cells, Chinese Medical Journal / Zhonghua Yixue Zazhi Yingwen Ban, Chinese Medical Association, Beijing, CN, vol. 124, no. 9, 30 April 2011, pages 1357-1361.
36. Chen S H et al., Rejection of disseminated metastases of colon carcinoma by synergism of IL-12 gene therapy and 4-1BB costimulation, Molecular Therapy: The Journal of the American Society of Gene Therapy, Academic Press; Nature Publishing Group, US, vol. 2, no. 1, 30 June 2000.
37. Rodrigo Garzón Manuel et al., Use of gene therapy in a subcutaneous murine model of lung cancer, Archivos De Bronconeumologia, Doyma, Barcelona, ES, vol. 42, no. 10, 30 September 2006.
38. Caruso M et al., Adenovirus-Mediated Interleukin-12 Gene Therapy For Metastatic Colon Carcinoma, Proceedings Of The National Academy Of Sciences, National Academy Of Sciences, US, vol. 93, 1 January 1996, pages 11302-11306.
39. Siobhan M Cashman et al., Inhibition of Choroidal Neovascularization by Adenovirus-Mediated Delivers of Short Hairpin RNAs Targeting VEGF as a Potential Therapy for AMD, Investigative Ophthalmology & Visual Science, vol. 47. no. 8, 1 August 2006, pages 3496-3504.
40. DATABASE WPI, Week 201004, Thomson Scientific, London, GB; AN 2010-A23395 & CN 101 596 316 A, 9 December 2009
41. KR 2009 0007067 A

<110> Industry-university Cooperation Foundation Hanyang University
<120> Compositions for enhancing antitumor immunity comprising an oncolytic adenovirus co-expressing interleukin-12 and shVEGF
<130> X16U10C0204
<150> KR 10-2015-0123644
   <151> 2015-09-01
<160> 6
<170> KoPatentIn 3.0
<210> 1
   <211> 648
   <212> DNA
   <213> Murine IL-12 mRNA sequence - mIL-12 (p35)
<400> 1
   atgtgtcaat cacgctacct cctctttttg gccacccttg ccctcctaaa ccacctcagt      60
   ttggccaggg tcattccagt ctctggacct gccaggtgtc ttagccagtc ccgaaacctg     120
   ctgaagacca cagatgacat ggtgaagacg gccagagaaa aactgaaaca ttattcctgc     180
   actgctgaag acatcgatca tgaagacatc acacgggacc aaaccagcac attgaagacc     240
   tgtttaccac tggaactaca caagaacgag agttgcctgg ctactagaga gacttcttcc     300
   acaacaagag ggagctgcct gcccccacag aagacgtctt tgatgatgac cctgtgcctt     360
   ggtagcatct atgaggactt gaagatgtac cagacagagt tccaggccat caacgcagca     420
   cttcagaatc acaaccatca gcagatcatt ctagacaagg gcatgctggt ggccatcgat     480
   gagctgatgc agtctctgaa tcataatggc gagactctgc gccagaaacc tcctgtggga     540
   gaagcagacc cttacagagt gaaaatgaag ctctgcatcc tgcttcacgc cttcagcacc     600
   cgcgtcgtga ccatcaacag ggtgatgggc tatctgagct ccgcctga                  648
<210> 2
   <211> 1008
   <212> DNA
   <213> Murine IL-12 mRNA sequence - mIL-12 (p40)
<400> 2
   atgtgtcctc agaagctaac catctcctgg tttgccatcg ttttgctggt gtctccactc      60
   atggccatgt gggagctgga gaaagacgtt tatgttgtag aggtggactg gactcccgat     120
   gcccctggag aaacagtgaa cctcacctgt gacacgcctg aagaagatga catcacctgg     180
   acctcagacc agagacatgg agtcataggc tctggaaaga ccctgaccat cactgtcaaa     240
   gagtttctag atgctggcca gtacacctgc cacaaaggag gcgagactct gagccactca     300
   catctgctgc tccacaagaa ggaaaatgga atttggtcca ctgaaatttt aaaaaatttc     360
   aaaaacaaga ctttcctgaa gtgtgaagca ccaaattact ccggacggtt cacgtgctca     420
   tggctggtgc aaagaaacat ggacttgaag ttcaacatca agagcagtag cagttcccct     480
   gactctcggg cagtgacatg tggaatggcg tctctgtctg cagagaaggt cacactggac     540
   caaagggact atgagaagta ttcagtgtcc tgccaggagg atgtcacctg cccaactgcc     600
   gaggagaccc tgcccattga actggcgttg gaagcacggc agcagaataa atatgagaac     660
   tacagcacca gcttcttcat cagggacatc atcaaaccag acccgcccaa gaacttgcag     720
   atgaagcctt tgaagaactc acaggtggag gtcagctggg agtaccctga ctcctggagc     780
   actccccatt cctacttctc cctcaagttc tttgttcgaa tccagcgcaa gaaagaaaag     840
   atgaaggaga cagaggaggg gtgtaaccag aaaggtgcgt tcctcgtaga gaagacatct     900
   accgaagtcc aatgcaaagg cgggaatgtc tgcgtgcaag ctcaggatcg ctattacaat     960
   tcctcatgca gcaagtgggc atgtgttccc tgcagggtcc gatcctag                 1008
<210> 3
   <211> 441
   <212> DNA
   <213> Murine VEGF mRNA sequence
<400> 3
   atgaactttc tgctctcttg ggtgcactgg accctggctt tactgctgta cctccaccat      60
   gccaagtggt cccaggctgc acccacgaca gaaggagagc agaagtccca tgaagtgatc     120
   aagttcatgg atgtctacca gcgaagctac tgccgtccga ttgagaccct ggtggacatc     180
   ttccaggagt accccgacga gatagagtac atcttcaagc cgtcctgtgt gccgctgatg     240
   cgctgtgcag gctgctgtaa cgatgaagcc ctggagtgcg tgcccacgtc agagagcaac     300
   atcaccatgc agatcatgcg gatcaaacct caccaaagcc agcacatagg agagatgagc     360
   ttcctacagc acagcagatg tgaatgcaga ccaaagaaag acagaacaaa gccagaaaaa     420
   tgtgacaagc caaggcggtg a                                               441
<210> 4
   <211> 71
   <212> DNA
   <213> Artificial Sequence
<220>
   <223> shVEGF sense
<400> 4
   gatcccggaa ggagagcaga agtcccatgt tcaagagaca tgggacttct gctctccttt          60
   ttttttggaa a                                                                71
<210> 5
   <211> 71
   <212> DNA
   <213> Artificial Sequence
<220>
   <223> shVEGF antisense
<400> 5
   tttccaaaaa aaaaggagag cagaagtccc atgtctcttg aacatgggac ttctgctctc          60
   cttccgggat c                                                               71
<210> 6
   <211> 640
   <212> DNA
   <213> Homo sapiens vascular endothelial growth factor (VEGF) mRNA
<400> 6
   tcgggcctcc gaaaccatga actttctgct gtcttgggtg cattggagcc ttgccttgct          60
   gctctacctc caccatgcca agtggtccca ggctgcaccc atggcagaag gaggggggca         120
   gaatcatcac gaagtggtga agttcatgga tgtctatcag cgcagctact gccatccaat         180
   cgagaccctg gtggacatct tccaggagta ccctgatgag atcgagtaca tcttcaagcc         240
   atcctgtgtg cccctgatgc gatgcggggg ctgctgcaat gacgagggcc tggagtgtgt         300
   gcccactgag gagtccaaca tcaccatgca gattatgcgg atcaaacctc accaaggcca         360
   gcacatagga gagatgagct tcctacagca caacaaatgt gaatgcagac caaagaaaga         420
   tagagcaaga caagaaaatc cctgtgggcc ttgctcagag cggagaaagc atttgtttgt         480
   acaagatccg cagacgtgta aatgttcctg caaaaacaca gactcgcgtt gcaaggcgag         540
   gcagcttgag ttaaacgaac gtacttgcag atgtgacaag ccgaggcggt gagccgggca         600
   ggaggaagga gcctccctca gggtttcggg aaccagatct                               640

## Claims

1. A recombinant adenovirus, comprising:
(i) an interleukin (IL-12) gene having at least one of an IL-12A (p35) gene sequence and an IL-12B (p40) gene sequence, and
(ii) a small hairpin RNA (shRNA) gene which is complementary to VEGF (vascular endothelial growth factor) mRNA, and suppresses VEGF gene expression.

2. The recombinant adenovirus of claim 1, wherein the IL-12 and shVEGF genes are inserted into E1 and E3 regions of the adenovirus, respectively.

3. The recombinant adenovirus of claim 1, wherein the recombinant adenovirus has an E1A region, but does not have an E1B region, which is deleted.

4. The recombinant adenovirus of claim 1, wherein the IL-12 gene includes an IL-12A (p35) gene sequence, an internal ribosome entry site (IRES) sequence and an IL-12B (p40) gene sequence.

5. A composition for use in a method of enhancing antitumor immunity, comprising:
(a) a therapeutically effective amount of a recombinant adenovirus comprising (i) an interleukin (IL-12) gene having at least one of an IL-12A (p35) gene sequence and an IL-12B (p40) gene sequence, and (ii) a small hairpin RNA (shRNA) gene which is complementary to VEGF (vascular endothelial growth factor) mRNA, and suppresses VEGF gene expression; and
(b) a pharmaceutically acceptable carrier.

6. The composition for use of claim 5, wherein the composition for enhancing antitumor immunity is intratumorally administered.

7. The composition for use of claim 5, wherein the composition increases a Th1/Th2 cytokine ratio in tumors.

8. An anticancer composition, comprising:
(a) a therapeutically effective amount of a recombinant adenovirus comprising (i) an interleukin (IL-12) gene having at least one of an IL-12A (p35) gene sequence and an IL-12B (p40) gene sequence, and (ii) a small hairpin RNA (shRNA) gene which is complementary to VEGF (vascular endothelial growth factor) mRNA, and suppresses VEGF gene expression; and
(b) a pharmaceutically acceptable carrier.

9. The anticancer composition of claim 8 for use in a method of treating a cancer, wherein the cancer is gastric cancer, lung cancer, breast cancer, ovarian cancer, liver cancer, bronchial cancer, nasopharyngeal cancer, laryngeal cancer, pancreatic cancer, bladder cancer, colorectal cancer, colon cancer, cervical cancer, brain cancer, prostate cancer, bone cancer, head and neck cancer, skin cancer, kidney cancer, polyploid carcinoma, thyroid cancer, parathyroid cancer or ureter cancer.

10. A composition for use in a method of preventing or treating tumor-induced thymic atrophy, comprising:
(a) a therapeutically effective amount of a recombinant adenovirus comprising (i) an interleukin (IL-12) gene having at least one of an IL-12A (p35) gene sequence and an IL-12B (p40) gene, and (ii) a small hairpin RNA (shRNA) gene which is complementary to VEGF (vascular endothelial growth factor) mRNA, and suppresses VEGF gene expression; and
(b) a pharmaceutically acceptable carrier.

11. The anticancer composition of claim 8 for use in a method of treating cancer.

12. The recombinant adenovirus of claim 1 for use in a method of treating cancer.

13. The recombinant adenovirus of claim 1 for use in a method of enhancing antitumor immunity.

14. The recombinant adenovirus of claim 1 for use in a method of preventing or treating tumor-induced thymic atrophy.

## Patentansprüche

1. Ein rekombinantes Adenovirus, umfassend:
(i) ein Interleukin (IL-12)-Gen mit mindestens einer von einer IL-12A (p35)-Gensequenz und einer IL-12B (p40)-Gensequenz, und
(ii) ein small hairpin RNA (shRNA)-Gen, das komplementär zu VEGF (vaskulärer endothelialer Wachstumsfaktor) mRNA ist und die VEGF-Genexpression unterdrückt.

2. Rekombinantes Adenovirus nach Anspruch 1, wobei die IL-12- und shVEGF-Gene in die E1- bzw. E3-Regionen des Adenovirus eingefügt sind.

3. Rekombinantes Adenovirus nach Anspruch 1, wobei das rekombinante Adenovirus eine E1A-Region hat, aber keine E1B-Region hat, die deletiert ist.

4. Rekombinantes Adenovirus nach Anspruch 1, wobei das IL-12-Gen eine IL-12A (p35)-Gensequenz, eine internal ribosome entry site (IRES) und eine IL-12B (p40)-Gensequenz enthält.

5. Eine Zusammensetzung zur Verwendung in einem Verfahren zur Steigerung der Antitumorimmunität, umfassend:
(a) eine therapeutisch wirksame Menge eines rekombinanten Adenovirus, umfassend (i) ein Interleukin (IL-12)-Gen mit mindestens einer von einer IL-12A (p35)-Gensequenz und einer IL-12B (p40)-Gensequenz, und (ii) ein small hairpin RNA (shRNA)-Gen, das komplementär zu VEGF (vaskulärer endothelialer Wachstumsfaktor) mRNA ist und die VEGF-Genexpression unterdrückt; und
(b) einen pharmazeutisch akzeptablen Träger.

6. Zusammensetzung zur Verwendung nach Anspruch 5, wobei die Zusammensetzung zur Verstärkung der Antitumorimmunität intratumoral verabreicht wird.

7. Zusammensetzung zur Verwendung nach Anspruch 5, wobei die Zusammensetzung ein Thl/Th2-Zytokin-Verhältnis in Tumoren erhöht.

8. Eine Antikrebszusammensetzung, umfassend:
(a) eine therapeutisch wirksame Menge eines rekombinanten Adenovirus, umfassend (i) ein Interleukin (IL-12)-Gen mit mindestens einer von einer IL-12A (p35)-Gensequenz und einer IL-12B (p40)-Gensequenz, und (ii) ein small hairpin RNA (shRNA)-Gen, das komplementär zu VEGF (vaskulärer endothelialer Wachstumsfaktor) mRNA ist und die VEGF-Genexpression unterdrückt; und
(b) einen pharmazeutisch akzeptablen Träger.

9. Antikrebszusammensetzung nach Anspruch 8 zur Verwendung in einem Verfahren zur Behandlung eines Krebses, wobei der Krebs Magenkrebs, Lungenkrebs, Brustkrebs, Eierstockkrebs, Leberkrebs, Bronchialkrebs, Nasopharynxkrebs, Kehlkopfkrebs, Bauchspeicheldrüsenkrebs, Blasenkrebs, kolorektaler Krebs, Dickdarmkrebs, Gebärmutterhalskrebs, Gehirnkrebs, Prostatakrebs, Knochenkrebs, Kopf- und Halskrebs, Hautkrebs, Nierenkrebs, polyploides Karzinom, Schilddrüsenkrebs, Nebenschilddrüsenkrebs oder Harnleiterkrebs ist.

10. Eine Zusammensetzung zur Verwendung in einem Verfahren zur Verhinderung oder Behandlung von Tumor-induzierter Thymusatrophie, umfassend:
(a) eine therapeutisch wirksame Menge eines rekombinanten Adenovirus, umfassend (i) ein Interleukin (IL-12)-Gen mit mindestens einer von einer IL-12A (p35)-Gensequenz und einem IL-12B (p40)-Gen, und (ii) ein small hairpin RNA (shRNA)-Gen, das komplementär zu VEGF (vaskulärer endothelialer Wachstumsfaktor) mRNA ist und die VEGF-Genexpression unterdrückt; und
(b) einen pharmazeutisch akzeptablen Träger.

11. Antikrebszusammensetzung nach Anspruch 8 zur Verwendung in einem Verfahren zur Behandlung von Krebs.

12. Rekombinantes Adenovirus nach Anspruch 1 zur Verwendung in einem Verfahren zur Behandlung von Krebs.

13. Rekombinantes Adenovirus nach Anspruch 1 zur Verwendung in einem Verfahren zur Steigerung der Antitumorimmunität.

14. Rekombinantes Adenovirus nach Anspruch 1 zur Verwendung in einem Verfahren zur Vorbeugung oder Behandlung von Tumor-induzierter Thymusatrophie.

## Revendications

1. Adénovirus recombinant, comprenant :
(i) un gène d'interleukine (IL-12) comprenant au moins une séquence de gène d'IL-12A (p35) et une séquence de gène d'IL-12B (p40), et
(ii) un gène de petit ARN en épingle à cheveu (ARNsh) qui est complémentaire de l'ARNm de VEGF (facteur de croissance endothéliale vasculaire), et supprime l'expression de gène de VEGF).

2. Adénovirus recombinant selon la revendication 1, dans lequel les gènes d'IL-12 et de shVEGF sont insérés dans des régions E1 et E3 de l'adénovirus, respectivement.

3. Adénovirus recombinant selon la revendication 1, dans lequel l'adénovirus recombinant a une région E1A, mais n'a pas de région E1B, qui est délétée.

4. Adénovirus recombinant selon la revendication 1, dans lequel le gène d'IL-12 inclut une séquence de gène d'IL-12A (p35), et une séquence de sites d'entrée interne des ribosomes (IRES) et une séquence de gène d'IL-12B (p40).

5. Composition pour utilisation dans un procédé de renforcement de l'immunité antitumorale, comprenant :
(a) une quantité thérapeutiquement efficace d'un adénovirus recombinant comprenant (i) un gène d'interleukine (IL-12) comportant au moins l'une d'une séquence de gène d'IL-12A (p35) et d'une séquence de gène d'IL-12B (p40), et (ii) un gène de petit ARN en épingle à cheveu (ARNsh) qui est complémentaire de l'ARNm de VEGF (facteur de croissance endothéliale vasculaire), et supprime l'expression de gène de VEGF ; et
(b) un support pharmaceutiquement acceptable.

6. Composition pour utilisation selon la revendication 5, dans laquelle la composition destinée à renforcer l'immunité antitumorale est administrée par voie intratumorale.

7. Composition pour utilisation selon la revendication 5, dans laquelle la composition augmente un rapport des cytokines Th1/Th2 dans les tumeurs.

8. Composition anticancéreuse, comprenant :
(a) une quantité thérapeutiquement efficace d'un adénovirus recombinant comprenant (i) un gène d'interleukine (IL-12) d'au moins l'une d'une séquence de gène d'IL-12A (p35) et d'une séquence de gène d'IL-12B (p40), et (ii) un gène de petit ARN en épingle à cheveu (ARNsh) qui est complémentaire de l'ARNm de VEGF (facteur de croissance endothéliale vasculaire), et supprime l'expression de gène de VEGF ; et
(b) un support pharmaceutiquement acceptable.

9. Composition anticancéreuse selon la revendication 8 pour utilisation dans un procédé de traitement d'un cancer, dans laquelle le cancer est un cancer gastrique, cancer des poumons, cancer du sein, cancer de l'ovaire, cancer du foie, cancer bronchique, cancer du nasopharynx, cancer du larynx, cancer du pancréas, cancer de la vessie, cancer colorectal, cancer du côlon, cancer du col de l'utérus, cancer du cerveau, cancer de la prostate, cancer des os, cancer de la tête et du cou, cancer de la peau, cancer du rein, carcinome polyploïde, cancer de la thyroïde, cancer de la parathyroïde ou cancer de l'uretère.

10. Composition pour utilisation dans un procédé de prévention ou de traitement d'une atrophie thymique induite par une tumeur, comprenant :
(a) une quantité thérapeutiquement efficace d'un adénovirus recombinant comprenant (i) un gène d'interleukine (IL-12) d'au moins l'une d'une séquence de gène d'IL-12A (p35) et d'une séquence de gène d'IL-12B (p40), et (ii) un gène de petit ARN en épingle à cheveu (ARNsh) qui est complémentaire de l'ARNm de VEGF (facteur de croissance endothéliale vasculaire), et supprime l'expression de gène de VEGF ; et
(b) un support pharmaceutiquement acceptable.

11. Composition anticancéreuse selon la revendication 8, pour utilisation dans un procédé de traitement du cancer.

12. Adénovirus recombinant selon la revendication 1, pour utilisation dans un procédé de traitement du cancer.

13. Adénovirus recombinant selon la revendication 1, pour utilisation dans un procédé de renforcement de l'immunité antitumorale.

14. Adénovirus recombinant selon la revendication 1, pour utilisation dans un procédé de prévention ou de traitement d'une atrophie thymique induite par tumeur.
